# EUROPEAN PATENT APPLICATION

(11) **EP 4 368 209 A1**
(43) Date of publication of application: **15.05.2024**
(21) Application number: 22206431.3
(22) Date of filing: 09.11.2022
(51) Int. Cl.: A61K 48/00, C12N 15/86, C12N 15/861

(54) **CARDIAC VASCULAR ENDOTHELIAL CELL-DERIVED NUCLEIC ACID REGULATORY ELEMENTS AND METHODS AND USE THEREOF**

(71) Applicant: Vrije Universiteit Brussel, 1050 Brussel (BE)
(72) Inventor: CHUAH, Lay, Kim, 3360 Bierbeek (BE); VANDENDRIESSCHE, Thierry, 3360 Bierbeek (BE)
(74) Representative: De Clercq & Partners

(57) **Abstract**

The present invention relates to nucleic acid regulatory elements that are able to enhance endothelial cell-targeted expression of genes, methods employing these regulatory elements and uses of these elements. Expression cassettes and vectors containing these nucleic acid regulatory elements are also disclosed. The present invention is particularly useful for applications using gene therapy, more particularly endothelium-directed gene therapy.

## Description

### FIELD

The present invention relates to nucleic acid regulatory elements that are able to enhance endothelium-targeted expression of genes, methods employing these regulatory elements and uses thereof. The invention further encompasses expression cassettes, vectors and pharmaceutical compositions comprising these regulatory elements. The present invention is particularly useful for applications using gene therapy, more particularly endothelium-directed gene therapy.

### BACKGROUND

The endothelium has substantial potential as therapeutic target. For example, endothelium-targeted gene therapy to express therapeutically relevant genes in endothelial cells (ECs) may be useful for the treatment of a cardiovascular disease (CVD), particularly coronary heart disease (CHD) and heart failure (HF). CHD is the most common type of heart disease characterized by build-up of plaque in the heart arteries that could lead to a sudden heart attack (myocardial infarction) or to chronic ischemic cardiomyopathy. HF is a common consequence of CHD that develops when the heart muscle cannot pump sufficiently to meet the body's needs for blood and oxygen. Despite remarkable progress made by surgical and medical therapies, including resynchronization therapy and use of ventricular assist devices, the long-term survival of patients with HF remains poor. Hence, a paradigm shift is needed that translates new knowledge and new technologies towards effective and safe innovative treatments for CHD and HF.

Gene therapy to the heart is relatively inefficient due to limitations in gene delivery and gene expression. Moreover, immune responses specific for the therapeutic gene product curtail the efficiency of gene therapy applications directed towards the heart. Hence, there is a need for approaches that maximize (trans)gene expression in the heart such as in cardiac endothelial cells.

Endothelium-targeted gene therapy can be used to treat endothelial cell dysfunction pertaining to a cardiovascular disease such as but not limited to deep vein thrombosis, pulmonary embolism, ischemic or haemorrhagic stroke, heart failure, coronary heart disease, myocardial ischemia and peripheral artery disease.

Endothelium-targeted gene therapy to express therapeutically relevant genes encoding proteins or non-coding RNA (ncRNA) can also be used to treat other endothelial cell-related disorders. For example, corneal endothelial disorder affects the function of endothelial cells. Corneal endothelial dysfunction occurs when corneal endothelial cells (CECs) are dramatically lost resulting in vision loss. Other nonexhaustive examples of endothelial dysfunctions pertain to (mono)genetic diseases such as Fabry disease.

Furthermore, secreting therapeutic proteins from endothelial cells could impact on diseases that would benefit from increased expression of circulating therapeutic proteins. Endothelium-targeted gene therapy can for instance be used to express proteins that are secreted in the circulation such as clotting factors (e.g. factor VIII, factor IX or von Willebrand factor for gene therapy of hemophilia A, hemophilia B or von Willebrand's disease, respectively). FVIII protein is synthesized and secreted by liver sinusoidal endothelial cells (LSECs) (Shahani et al. 2014. J Thromb Haemost. 12:36-42), which have been suggested as target for gene therapy for hemophilia A (El-Maarri et al. 2020. Hamostaseologie 40:S26-S31).

Hence, there is a need to establish effective cures by gene therapy to enable robust expression of the cognate therapeutic genes in endothelial cells. This requires the development of potent expression cassettes containing the genes of interest. Consequently, there is a need to identify robust nucleic acid regulatory elements capable of substantially increasing transcription in the endothelium.

WO 2017/109039 A1 discloses endothelial cell-specific cis-regulatory elements (EC-CREs) that were identified based on expression data from endothelial cells in publicly available databases. Genes that were highly and specifically expressed in endothelial cells were selected for identification of regulatory elements in the sequence upstream of their transcription start site.

It is an object of the present invention to further increase the efficiency and safety of endothelium-directed gene therapy.

### SUMMARY

The present inventors used purified cardiac vascular endothelial cells, in particular human coronary artery endothelial cells (CAEC) and human cardiac microvascular endothelial cells (CMEC), to identify novel cis-regulatory elements (CREs), in particular endothelial cell-targeted CREs (EC-CREs). These regulatory elements were subsequently validated *in vivo* yielding high gene expression in endothelial cells, such as for instance in liver sinusoidal endothelial cells (LSECs). Combinations of the newly identified EC-CREs with EC-CREs from the prior art, in particular the nucleic acid regulatory element of SEQ ID NO:17 (IFI27-EC-CRE1b), could further increase endothelial cell-targeted expression of a transgene operably linked to it, in a synergistic way. The nucleic acid regulatory elements identified herein, allow for the use of lower and thus safer vector doses in gene therapy, more particularly endothelium-targeted gene therapy, while maximizing therapeutic efficacy.

The invention therefore provides the following aspects:
Aspect 1. An (isolated) nucleic acid regulatory element for enhancing endothelial cell-targeted gene expression, comprising, consisting essentially of, or consisting of a sequence selected from the group consisting of: SEQ ID NO:3, SEQ ID NO: 1, SEQ ID NO:2, and a sequence having at least 80%, preferably at least 85%, more preferably at least 90%, even more preferably at least 95%, such as 95%, 96%, 97%, 98%, or 99%, identity to (the full-length of) any one of these sequences (i.e. a sequence that has at least 80%, preferably at least 85%, more preferably at least 90%, even more preferably at least 95%, such as 95%, 96%, 97%, 98%, or 99%, identity to (the full-length of) the sequence set forth in SEQ ID NO:3, SEQ ID NO:1 or SEQ ID NO:2), or a functional fragment thereof. In preferred embodiments of this aspect, the nucleic acid regulatory element is maximal 600 nucleotides, preferably maximal 550 nucleotides long and comprises, consists essentially of, or consists of a sequence selected from the group consisting of: SEQ ID NO:3, SEQ ID NO: 1, SEQ ID NO:2, and a sequence having at least 80%, preferably at least 85%, more preferably at least 90%, even more preferably at least 95%, such as 95%, 96%, 97%, 98%, or 99%, identity to (the full-length of) any one of these sequences, or a functional fragment thereof.

In embodiments of said aspect, said nucleic acid regulatory element is maximal 500 nucleotides long, preferably maximal 450 nucleotides, more preferably maximal 425 nucleotides, and comprises, consists essentially of or consists of the sequence set forth in SEQ ID NO:3, or a sequence having at least 80%, preferably at least 85%, more preferably at least 90%, even more preferably at least 95%, such as 95%, 96%, 97%, 98%, or 99%, identity to (the full-length of) the sequence set forth in SEQ ID NO:3, or a functional fragment thereof. In a particular embodiment of said aspect, said regulatory element consists of the sequence set forth in SEQ ID NO:3 (i.e. is CV-EC-CRE18). In embodiments of said aspect, said nucleic acid regulatory element is maximal 500 nucleotides long, preferably maximal 450 nucleotides, and comprises, consists essentially of or consists of the sequence set forth in SEQ ID NO: 1, or a sequence having at least 80%, preferably at least 85%, more preferably at least 90%, even more preferably at least 95%, such as 95%, 96%, 97%, 98%, or 99%, identity to (the full-length of) the sequence set forth in SEQ ID NO:1, or a functional fragment thereof. In a particular embodiment of said aspect, said regulatory element consists of the sequence set forth in SEQ ID NO:1 (i.e. is CV-EC-CRE3). In embodiments of said aspect, said nucleic acid regulatory element is maximal 600 nucleotides long, preferably maximal 550 nucleotides and comprises, consists essentially of or consists of the sequence set forth in SEQ ID NO:2, or a sequence having at least 80%, preferably at least 85%, more preferably at least 90%, even more preferably at least 95%, such as 95%, 96%, 97%, 98%, or 99%, identity to (the full-length of) the sequence set forth in SEQ ID NO:2, or a functional fragment thereof. In a particular embodiment of said aspect, said regulatory element consists of the sequence set forth in SEQ ID NO:2 (i.e. is CV-EC-CRE9).

In embodiments of said aspect, the nucleic acid regulatory element comprises at least one, preferably all, transcription factor binding site selected from the group consisting of: a transcription factor binding site (TFBS) for POLR2A, a TFBS for YY1, a TFBS for ELF1, a TFBS for REST, a TFBS for EGR1, a TFBS for IRF1, a TFBS for JUND, a TFBS for RCOR1, a TFBS for PML, a TFBS for EP300 and a TFBS for CEBP.

Aspect 2. The nucleic acid regulatory element according to aspect 1, wherein said functional fragment is a functional fragment of a sequence selected from the group consisting of: SEQ ID NO:3, SEQ ID NO:1 and SEQ ID NO:2, and wherein said functional fragment comprises, consists essentially of, or consists of at least 20, preferably at least 25, more preferably at least 50, at least 100, at least 200 or at least 250, even more preferably at least 300, at least 350 or at least 400 contiguous nucleotides from the sequence from which it is derived.

In embodiments of said aspect, said nucleic acid regulatory element is a functional fragment of SEQ ID NO:3, wherein said functional fragment comprises or consists of at least 350, preferably at least 360, 370 or 380, more preferably at least 390 or 400 contiguous nucleotides from SEQ ID NO:3. In embodiments of said aspect, said nucleic acid regulatory element is a functional fragment of SEQ ID NO:1, wherein said functional fragment comprises or consists of at least 370, preferably at least 380, 390 or 400, more preferably at least 410 or 420 contiguous nucleotides from SEQ ID NO:1. In embodiments of said aspect, said nucleic acid regulatory element is a functional fragment of SEQ ID NO:2, wherein said functional fragment comprises or consists of at least 470, preferably at least 480, 490 or 500, more preferably at least 510 or 520 contiguous nucleotides from SEQ ID NO:2.

Aspect 3: A nucleic acid regulatory element for enhancing endothelial cell-targeted gene expression, comprising, consisting essentially of, or consisting of the complement of a sequence as defined in aspect 1 or 2.

Aspect 4. A nucleic acid regulatory element for enhancing endothelial cell-targeted gene expression, which is capable of hybridizing under stringent conditions to a sequence selected from the group consisting of: SEQ ID NO:3, SEQ ID NO:1, SEQ ID NO:2, and a sequence having at least 80%, preferably at least 85%, more preferably at least 90%, even more preferably at least 95%, such as 95%, 96%, 97%, 98%, or 99%, identity to (the full-length of) any one of these sequences, or a functional fragment thereof.

Aspect 5. The nucleic acid regulatory element according to any one of aspects 1 to 4, having a total length of 600 nucleotides or less, preferably 550 nucleotides or less.

Aspect 6: Use, preferably an *in vitro* or *ex vivo* use, of the nucleic acid regulatory element according to any one aspects 1 to 5 in a nucleic acid expression cassette or a vector, for enhancing gene expression in an endothelial cell.

Aspect 7. A nucleic acid expression cassette comprising at least one nucleic acid regulatory element according to any one of aspects 1 to 5, operably linked to a promoter.

Aspect 8. A nucleic acid expression cassette comprising at least one nucleic acid regulatory element according to any one of aspects 1 to 5, operably linked to a promoter and a transgene.

Aspect 9. The nucleic acid expression cassette according to aspect 7 or 8, further comprising an endothelial cell-targeted regulatory element comprising or consisting of the sequence set forth in SEQ ID NO:17 or a sequence having at least 95% identity to (the full-length of) the sequence set forth in SEQ ID NO:17.

Aspect 10. The nucleic acid expression cassette according to any one of aspects 7 to 9, wherein the promoter is an endothelial cell-targeted promoter, preferably an ICAM2 promoter such as the ICAM2 promoter defined by SEQ ID NO:18, a FVIII promoter such as the FVIII promoter defined by SEQ ID NO:19 or a EDN1 promoter such as the EDN1 promoter defined by SEQ ID NO:20, more preferably an ICAM2 promoter.

Aspect 11. The nucleic acid expression cassette according to any one of aspects 8 to 10, wherein the transgene encodes a therapeutic protein or an immunogenic protein.

Aspect 12. The nucleic acid expression cassette according to any one of aspects 8 to 11, wherein the transgene encodes a secretable protein or a non-secreted protein (such as a structural protein, an enzyme or a transcription factor).

Aspect 13. The nucleic acid expression cassette according to any one of aspects 8 to 10, wherein the transgene encodes a non-coding RNA, preferably a microRNA (miRNA), a long non-coding RNA (lncRNA), a circular RNA (cRNA), a short hairpin RNA (shRNA), an antisense RNA, a small interfering RNA (siRNA), an enhancer RNA (eRNA), a catalytic RNA, a guide RNA, or an RNA aptamer.

Aspect 14. The nucleic acid expression cassette according to any one of aspects 8 to 12, wherein the transgene encodes coagulation factor VIII (FVIII), coagulation factor VII (FVII), coagulation factor IX (FIX), coagulation factor XI (FXI), tissue factor (TF), von Willebrand factor (vWF), tissue factor pathway inhibitor (TFPI), a disintegrin and metalloproteinase with a thrombospondin type 1 motif, member 13 (ADAMTS13), hepatocyte growth factor (HGF), vascular endothelial growth factor (VEGF), placental growth factor (PLGF), fibroblast growth factor (FGF), soluble fms-like tyrosine kinase-1 (sFLT1), α1-antitrypsin (AAT), apolipoprotein A-I (apoA-I), a tissue inhibitor of metalloproteinase (TIMP, e.g. TIMP-3), an anti-VEGF antibody or an anti-PLGF antibody, erythropoietin, lipoprotein lipase, adiponectin, insulin, a nitric oxide synthase (NOS), or a hypoxia-inducible factor 1α (HIF-1α).

In particular embodiments of this aspect, the transgene encodes a coagulation factor VIII (FVIII).

Aspect 15. The nucleic acid expression cassette according to any one of aspects 7 to 14, further comprising an intron, preferably a Minute Virus of Mouse (MVM) intron, more preferably the MVM intron defined by SEQ ID NO:21.

Aspect 16. The nucleic acid expression cassette according to any one of aspects 7 to 15, further comprising a transcriptional termination signal such as the synthetic polyadenylation signal defined by SEQ ID NO22.

Aspect 17. A vector comprising the nucleic acid regulatory element according to any one of aspects 1 to 5, or the nucleic acid expression cassette according to any one of aspects 7 to 16.

Aspect 18. The vector according to aspect 17, which is a viral vector such as an adeno-associated viral (AAV) vector, a lentiviral (LV) vector or an adenoviral (AV) vector, preferably an AAV vector.

Aspect 19. The vector according to aspect 17, which is a non-viral vector, preferably a plasmid, a minicircle, an episomal vector, or a transposon-based vector such as a PiggyBac-based vector or a Sleeping Beauty-based vector.

Aspect 20. A pharmaceutical composition comprising the nucleic acid expression cassette according to any one of aspects 7 to 16, or the vector according to any one of aspects 17 to 19, and a pharmaceutically acceptable carrier.

Aspect 21. A nucleic acid regulatory element according to any one of aspects 1 to 5, a nucleic acid expression cassette according to any one of aspects 7 to 16, a vector according to any one of aspects 17 to 19, or a pharmaceutical composition according to aspect 20 for use in medicine.

A related aspect is directed to use of a nucleic acid regulatory element according to any one of aspects 1 to 5, a nucleic acid expression cassette according to any one of aspects 7 to 16 or a vector according to any one of aspects 17 to 19 for the manufacture of a medicament.

Aspect 22. A nucleic acid regulatory element according to any one of aspects 1 to 5, a nucleic acid expression cassette according to any one of aspects 7 to 16, a vector according to any one of aspects 17 to 19, or a pharmaceutical composition according to aspect 20 for use in gene therapy, preferably endothelium-directed gene therapy.

A related aspect is directed to use of a nucleic acid regulatory element according to any one of aspects 1 to 5, a nucleic acid expression cassette according to any one of aspects 7 to 16 or a vector according to any one of aspects 17 to 19 for the manufacture of a medicament for gene therapy, preferably endothelium-directed gene therapy.

A related aspect is directed to a method for gene therapy, in particular endothelium-directed gene therapy, in a subject in need of said gene therapy, comprising:
- introducing in the subject, in particular in an endothelial cell such as in a liver sinusoidal endothelial cell of the subject, a nucleic acid expression cassette according to any one of aspects 7 to 16, a vector according to any one of aspects 17 to 19, or a pharmaceutical composition according to aspect 20, wherein the nucleic acid expression cassette, the vector or the pharmaceutical composition comprises a nucleic acid regulatory element according to any one of aspects 1 to 5, operably linked to a promoter and a transgene; and
- expressing a therapeutically effective amount of the transgene product in the subject, in particular in the endothelial cell such as in the liver sinusoidal endothelial cell of the subject.

Aspect 23. A nucleic acid regulatory element according to any one of aspects 1 to 5, a nucleic acid expression cassette according to any one of aspects 7 to 16, a vector according to any one of aspects 17 to 19, or a pharmaceutical composition according to aspect 20 for use in the treatment of a disease or disorder selected from the group comprising: a cardiovascular disease or disorder, a disease or disorder associated with endothelial cell dysfunction, a blood clotting disorder, diabetes, insulin resistance, cancer, vascular inflammation, kidney disease, vascular leakage, hemorrhagic fever and autoimmune disease.

A related aspect is directed to a method for treating a disease or disorder selected from the group comprising: a cardiovascular disease or disorder, a disease or disorder associated with endothelial cell dysfunction, a blood clotting disorder, diabetes, insulin resistance, cancer, vascular inflammation, kidney disease, vascular leakage, hemorrhagic fever and autoimmune disease, in a subject in need of said treatment, said method comprising administering a therapeutically effective amount of a nucleic acid expression cassette according to any one of aspects 7 to 16, a vector according to any one of aspects 17 to 19, or a pharmaceutical composition according to aspect 20 to the subject.

A related aspect is directed to use of a nucleic acid expression cassette according to any one of aspects 7 to 16, a vector according to any one of aspects 17 to 19 for the manufacture of a medicament for the treatment of a disease or disorder selected from the group comprising: a cardiovascular disease or disorder, a disease or disorder associated with endothelial cell dysfunction, a blood clotting disorder, diabetes, insulin resistance, cancer, vascular inflammation, kidney disease, vascular leakage, hemorrhagic fever and autoimmune disease.

A particular embodiment is directed to the nucleic acid regulatory element, the nucleic acid expression cassette, the vector, or the pharmaceutical composition for use in the treatment of hemophilia A.

Aspect 24. A method, optionally an *in vitro* or *ex vivo* method, for expressing a transgene product in an endothelial cell comprising:
- introducing the nucleic acid expression cassette according to any one of aspects 8 to 16, or the vector according to any one of aspects 17 to 19 into the endothelial cell such as in a liver sinusoidal endothelial cell; and
- expressing the transgene product in the endothelial cell.

### BRIEF DESCRIPTION OF DRAWINGS

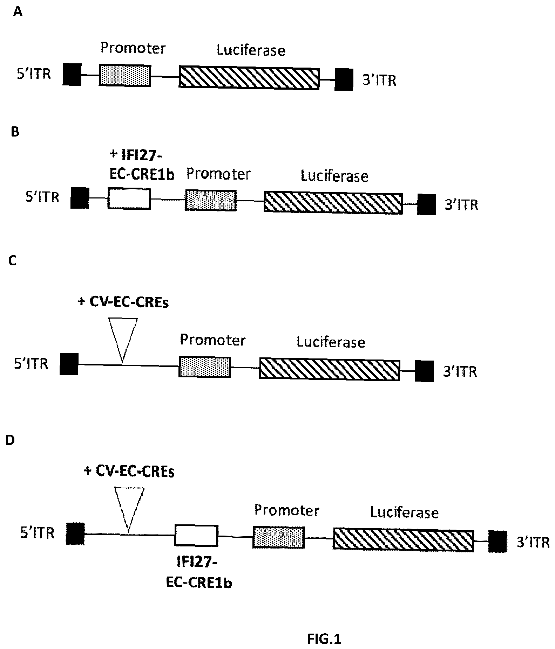

**FIG 1****:** Schematic representation of the AAV constructs. The promoter used was the endothelial-targeted promoter ICAM2, FVIII or EDN1 promoter. These AAV vectors were designed to express a luciferase reporter gene (*Luc2*) driven from the endothelial-targeted promoter operably linked to a regulatory element CV-EC-CRE as identified herein (C and D) or without a CV-EC-CRE used as control vectors (A and B). All vector constructs contained an MVM intron (not shown) downstream of the promoter and a synthetic polyadenylation site (pA) downstream of the *Luc2* reporter gene (not shown). ITR: AAV inverted terminal repeat. **(A)** AAVss-ICAM2-Luc-pA vector construct (SEQ ID NO:11): control vector without any cis-regulatory element; the ICAM2 promoter drives *Luc2* expression; **(B)** AAVss-IFI27-EC-CRE1b-ICAM2-Luc-pA (SEQ ID NO:4): this AAV vector contained one endothelial cell-specific cis-regulatory element designated as IFI27-EC-CRE1b operably linked to the ICAM2 promoter to drive *Luc2* expression; **(C)** AAVss-CV-EC-CRE-ICAM2-Luc-pA (e.g. AAVss-CV-EC-CRE18-ICAM2-Luc-pA (SEQ ID NO:10)): this AAV vector contained one cardiac vascular endothelial cell-derived cis-regulatory element (CV-EC-CRE), in particular CV-EC-CRE-18, operably linked to the ICAM2 promoter to drive *Luc2* expression. **(D)** AAVss-(CV-EC-CRE)-(IFI27-EC-CRE1b)-ICAM2-Luc-pA (e.g. AAVss-(CV-EC-CRE3)-(IFI27-EC-CRE1b)-ICAM2-Luc-pA (SEQ ID NO:5), AAVss-(CV-EC-CRE9)-(IFI27-EC-CRE1b)-ICAM2-Luc-pA (SEQ ID NO:6), AAVss-(CV-EC-CRE18)-(IFI27-EC-CRE1b)-ICAM2-Luc-pA (SEQ ID NO:7) and AAVss-(CV-EC-CRE16)-(IFI27-EC-CRE1b)-ICAM2-Luc-pA (SEQ ID NO:15)): these AAV vectors contained a combination of CV-EC-CRE (in particular CV-EC-CRE-3, CV-EC-CRE-9 , CV-EC-CRE-18 or CV-EC-CRE-16, respectively) and IFI27-EC-CRE1b operably linked to the ICAM2 promoter to drive *Luc2* expression.

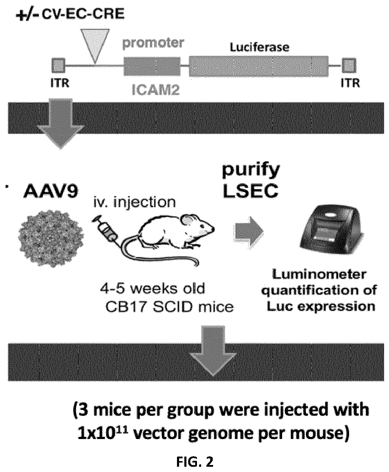

**FIG. 2****:** Validation of CV-EC-CREs in isolated liver sinusoidal endothelial cells (LSECs) after *in vivo* AAV-Luc transduction. AAV vectors with or without CV-EC-CREs were produced and injected intravenously into 4-5 weeks old CB-17 SCID mice with a dose of 1×10¹¹ vg per mouse; 5 months post injection, experimental mice were euthanized and LSECs were isolated and purified from the mouse livers. *Luc* gene expression was quantified using a luminometer.

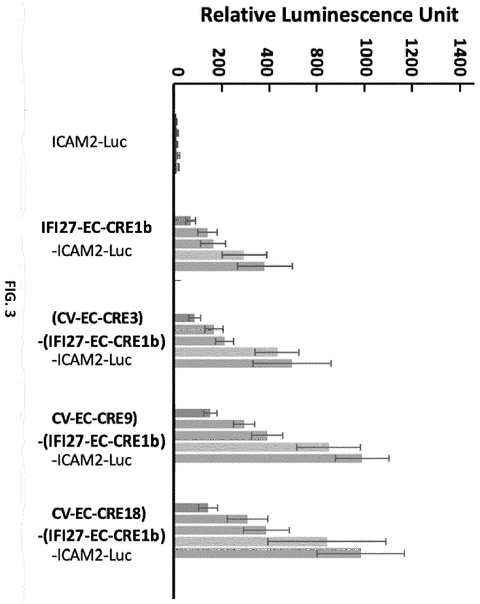

**FIG. 3*****:** In vitro* analysis of luciferase activity in LSEC purified from AAV9-transduced mice livers. Mice were injected with a dose corresponding to 10¹¹ vector genomes (vg) per mouse (i.v.). Each bar from left to right indicate the cell number (40000, 80000, 100000, 200000 and 300000 of purified LSEC cells) used for the measurement of luciferase activity of each construct. Luciferase activity was determined by luminometric analysis. The different AAV vector designs and CREs are indicated for each of the injected cohorts (n = 3 mice per cohort). Luciferase activity is expressed as relative luminescence units (RLU).

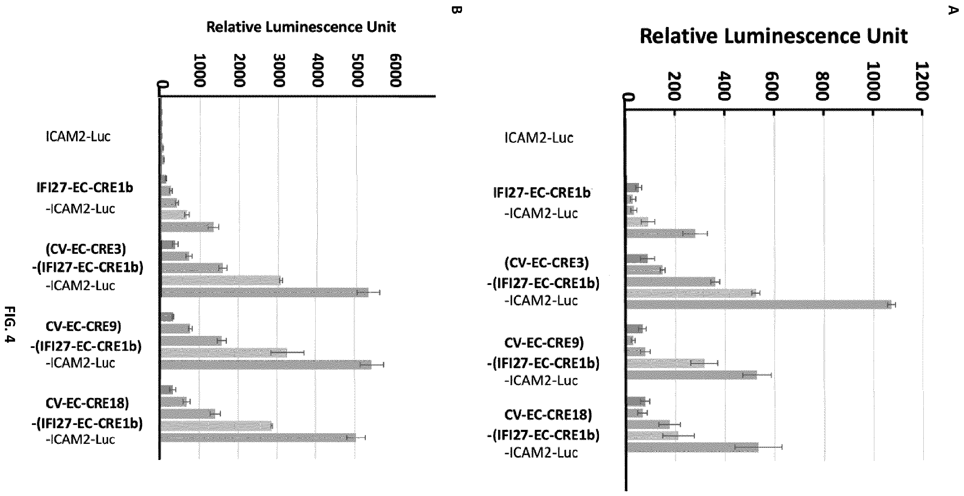

**FIG. 4****:** Vector dose-response analysis. Mice were injected with AAV9 vectors at a dose corresponding to 1×10¹¹ vector genomes (vg) **(A)** or 3×10¹¹ vg per mouse **(B)** and LSECs were purified from the AAV9-transduced mice livers. Each bar from left to right indicate the cell number from 20,000, 40,000, 80,000, 160,000 to 320,000 of purified LSEC cells used for the measurement of luciferase activity of each construct. Luciferase activity was determined by luminometric analysis. The different AAV vector designs and CREs are indicated for each of the injected cohorts (n = 4 mice per cohort). Luciferase activity is expressed as relative luminescence units (RLU).

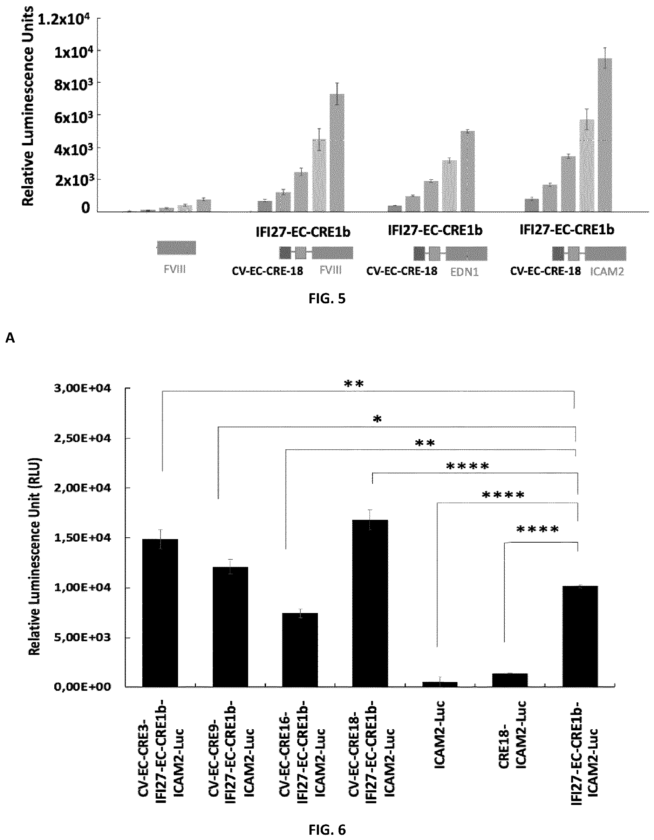

**FIG. 5****:** Validation of CV-EC-CREs *in vivo* using different promoters. Mice were injected with AAV9 vectors at a dose corresponding to 8×10¹¹ vg per mouse (i.v.). Luciferase activity was determined *in vitro* by luminometric analysis in LSECs isolated and purified from the AAV9-transduced mice livers Each bar from left to right indicate the cell number from 20000, 40000, 80000, 160000 to 320000 of purified LSEC cells used for the measurement of luciferase activity of each construct. The different AAV vector designs and CREs are indicated for each of the injected cohorts (n = 4 mice per cohort).

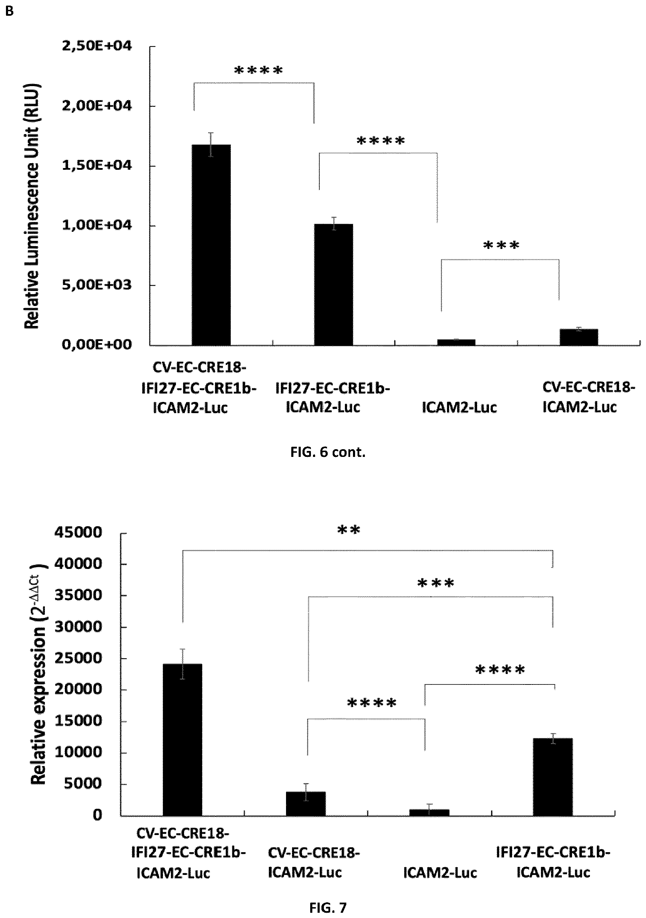

**FIG. 6****:** Analysis of luciferase activity in LSECs isolated and purified from AAV9-transduced mice livers.
Mice were injected i.v. with AAV9 vectors at a dose corresponding to 10¹² vector genomes (vg) per mouse. Luciferase activity was determined *in vitro* by luminometric analysis using 320,000 isolated LSECs per cohort. The expression of luciferase in the isolated LSEC transduced with different vectors was validated by Bioluminescence assay (ONE-Glo^{™} Luciferase Assay System, Promega, USA). The different AAV vector designs and CREs are indicated for each of the cohorts . Welch t-test were conducted to examine the significant difference among groups (n=6, except ICAM2 n=5). ^{∗}P<0.05, ^{∗∗}P<001, ^{∗∗∗}P<0.02, ^{∗∗∗∗}P<0.0001.
**FIG. 7****: mRNA expression of luciferase gene in LSECs isolated and purified from AAV9-transduced** mice. mRNA expression of luciferase gene in the isolated and purified LSECs from Fig. 6 was measured by quantitative reverse transcriptase PCR. Welch's t test were conducted to examine the significant difference among groups (n=4 except for ICAM2, n=5). ^{∗∗}P<001, ^{∗∗∗}P<0.02, ^{∗∗∗∗}P< 0.001.

### DESCRIPTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of' and "consisting essentially of', which enjoy well-established meanings in patent terminology.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

The terms or definitions provided herein are provided to aid in the understanding of the invention. Unless specifically defined herein, all terms used herein have the same meaning as they would to one skilled in the art of the present invention.

For general methods relating to the invention, reference is made *inter alia* to well-known textbooks, including, e.g., "Molecular Cloning: A Laboratory Manual, 4th Ed." (Green and Sambrook, 2012, Cold Spring Harbor Laboratory Press) and "Current Protocols in Molecular Biology" VoIs. 1-4, (Ausubel et al., 2001, John Wiley and Sons, Inc. New York).

In an aspect, the invention relates to a nucleic acid regulatory element, in particular an isolated nucleic acid regulatory element, for enhancing endothelial cell-targeted gene expression comprising, consisting essentially of (i.e., the regulatory element may for instance additionally comprise sequences used for cloning purposes, but the indicated sequences make up the essential part of the regulatory element, e.g. they do not form part of a larger regulatory region such as a (homologous) promoter), or consisting of a sequence selected from the group consisting of: SEQ ID NO:3, SEQ ID NO:1, SEQ ID NO:2, and a sequence having at least 80%, preferably at least 85%, more preferably at least 90%, even more preferably at least 95%, such as 95%, 96%, 97%, 98%, or 99%, identity to (the full-length of) any one of these sequences, or a functional fragment thereof (i.e. a functional fragment of a sequence selected from the group consisting of: SEQ ID NO:3, SEQ ID NO:1, SEQ ID NO:2, and a sequence having at least 80%, preferably at least 85%, more preferably at least 90%, even more preferably at least 95%, such as 95%, 96%, 97%, 98%, or 99%, identity to (the full-length of) any one of these sequences, and still performing the function of said regulatory element, i.e. increasing endothelial cell-targeted gene expression).

In certain embodiments, the nucleic acid regulatory element described herein consists of a sequence selected from the group consisting of: SEQ ID NO:3, SEQ ID NO:1, and SEQ ID NO:2, and at least one flanking nucleotide sequence (i.e. a 5' flanking nucleotide sequence and/or a 3' flanking nucleotide sequence), wherein said at least one flanking nucleotide sequence has a maximal length of 50 nucleotides, preferably a maximal length of 45, 40, 35, 30 or 25 nucleotides, more preferably a maximal length of 20, 19, 18, 17, 16, 15, 14, 13, 12, 11, 10, 9, 8, 7, 6, 5, 4, 3, 2 or 1 nucleotides. In certain embodiments, the nucleic acid regulatory element described herein consists of a sequence selected from the group consisting of: SEQ ID NO:3, SEQ ID NO:1 and SEQ ID NO:2, and two flanking nucleotide sequences located on opposite ends of said sequence selected from the group consisting of: SEQ ID NO:3, SEQ ID NO:1 and SEQ ID NO:2 (or a 5' flanking nucleotide sequence and a 3' flanking nucleotide sequence), wherein each flanking nucleotide sequence independently has a maximal length of 50 nucleotides, preferably a maximal length of 45, 40, 35, 30 or 25 nucleotides, more preferably a maximal length of 20, 19, 18, 17, 16, 15, 14, 13, 12, 11, 10, 9, 8, 7, 6, 5, 4, 3, 2 or 1 nucleotides. In embodiments, the flanking nucleotide sequence is a heterologous sequence (e.g. a sequence used for cloning purposes). In other embodiments, the flanking nucleotide sequence is a homologous sequence.

Within the meaning of the present invention, "heterologous" in relation to a given sequence is understood as meaning any nucleic acid sequence other than those which are immediately adjacent to the said sequence in nature. The term "homologous" as used herein in relation to a given sequence is understood as meaning a nucleic acid sequence which is immediately adjacent to the said sequence in nature.

A "nucleic acid regulatory element" or "regulatory element", also called "CRE" (cis-regulatory element), "CRM" (cis-regulatory module), or "EC-CRE" (endothelial cell-targeted cis-regulatory element or endothelium-targeted cis-regulatory elemnt) or "CV-EC-CRE" (cardiac vascular endothelial cell-derived cis-regulatory element) as used herein refers to a transcriptional control element, in particular a non-coding cis-acting transcriptional control element, capable of regulating and/or controlling transcription of a gene, in particular tissue- or cell-targeted transcription of a gene, more particularly endothelium- or endothelial cell-targeted expression of a gene. Regulatory elements comprise at least one transcription factor binding site (TFBS), in particular at least one binding site for a tissue- or cell-targeted transcription factor, more in particular at least one binding site for an endothelium- or endothelial cell-targeted transcription factor. Typically, regulatory elements as used herein increase or enhance promoter-driven gene expression when compared to the transcription of the gene from the promoter alone, without the regulatory elements. Thus, regulatory elements particularly comprise enhancer sequences, although it is to be understood that the regulatory elements enhancing transcription are not limited to typical far upstream enhancer sequences, but may occur at any distance of the gene they regulate. Indeed, it is known in the art that sequences regulating transcription may be situated either upstream (e.g. in the promoter region) or downstream (e.g. in the 3'UTR) of the gene they regulate *in vivo,* and may be located in the immediate vicinity of the gene or further away or even within the gene itself. Of note, although regulatory elements as disclosed herein typically comprise naturally occurring sequences, combinations of (parts of) such regulatory elements or several copies of a regulatory element, i.e. regulatory elements comprising non-naturally occurring sequences, are themselves also envisaged as regulatory element. Regulatory elements as used herein may comprise part of a larger sequence involved in transcriptional control, e.g. part of a promoter sequence. However, regulatory elements alone are typically not sufficient to initiate transcription, but require a promoter to this end. The regulatory elements disclosed herein are provided as nucleic acid molecules, i.e. isolated nucleic acids, or isolated nucleic acid molecules. Said nucleic acid regulatory element hence have a sequence which is only a small part of the naturally occurring genomic sequence and hence is not naturally occurring as such, but is isolated therefrom.

The term "nucleic acid" as used herein typically refers to an oligomer or polymer (preferably a linear polymer) of any length composed essentially of nucleotides. A nucleotide unit commonly includes a heterocyclic base, a sugar group, and at least one, e.g. one, two, or three, phosphate groups, including modified or substituted phosphate groups. Heterocyclic bases may include inter alia purine and pyrimidine bases such as adenine (A), guanine (G), cytosine (C), thymine (T) and uracil (U) which are widespread in naturally-occurring nucleic acids, other naturally-occurring bases (e.g., xanthine, inosine, hypoxanthine) as well as chemically or biochemically modified (e.g., methylated), non-natural or derivatised bases. Sugar groups may include inter alia pentose (pentofuranose) groups such as preferably ribose and/or 2-deoxyribose common in naturally-occurring nucleic acids, or arabinose, 2-deoxyarabinose, threose or hexose sugar groups, as well as modified or substituted sugar groups. Nucleic acids as intended herein may include naturally occurring nucleotides, modified nucleotides or mixtures thereof. A modified nucleotide may include a modified heterocyclic base, a modified sugar moiety, a modified phosphate group or a combination thereof. Modifications of phosphate groups or sugars may be introduced to improve stability, resistance to enzymatic degradation, or some other useful property. The term "nucleic acid" further preferably encompasses DNA, RNA and DNA/RNA hybrid molecules, specifically including hnRNA, pre-mRNA, guide(g) RNA, mRNA, cDNA, non-coding (nc)-RNA, long non-coding RNA (lnc)RNA, short-hairpin (sh)RNA, small interfering (si)RNA, micro(mi)RNA, circular (c)RNA, genomic DNA, amplification products, oligonucleotides, and synthetic (e.g., chemically synthesised) DNA, RNA or DNA/RNA hybrids. A nucleic acid can be naturally occurring, e.g., present in or isolated from nature; or can be non-naturally occurring, e.g., recombinant, i.e., produced by recombinant DNA technology, and/or partly or entirely, chemically or biochemically synthesised. A "nucleic acid" can be double-stranded, partly double stranded, or single-stranded. Where single-stranded, the nucleic acid can be the sense strand or the antisense strand. In addition, nucleic acid can be circular or linear.

As used herein "transcription factor binding site", "transcription factor binding sequence" or "TFBS" refers to a sequence of a nucleic acid region to which transcription factors bind. Non-limiting examples of TFBS include binding sites for RNA polymerase II, subunit A, also known as POLR2A; YY1 Transcription Factor; E74 Like ETS Transcription Factor 1, also known as ELF1; RE1 Silencing Transcription Factor, also known as REST; Early Growth Response 1, also known as EGR1; Interferon Regulatory Factor 1, also known as IRF1, IRF-1 or MAR; JunD Proto-Oncogene, AP-1 Transcription Factor Subunit, also known as JUND; REST Corepressor 1, also known as RCOR1; PML Nuclear Body Scaffold, also known as PML; E1A Binding Protein P300 or histone acetyltransferase P300, also known as EP300 or P300; E-box binding proteins E47 and E12, CCAAT/enhancer-binding protein, also known as CEB/P, C/EPB or CEPB. Transcription factor binding sites may be found in databases such as Transfac^{®}.

As used herein "endothelium-targeted expression" or "endothelial cell-targeted expression" refers to the preferential or predominant expression of a (trans)gene (as RNA and/or polypeptide) in endothelial cells or in endothelial tissue or endothelium comprising endothelial cells, as compared to other (i.e. non-endothelial) cells and tissues. According to particular embodiments, at least 50%, more particularly at least 60%, at least 70%, at least 75%, at least 80%, at least 85%, at least 90%, at least 95%, at least 98%, at least 99% or 100% of the (trans)gene expression occurs within endothelial cells or tissue. According to a particular embodiment, endothelium-targeted expression entails that there is less than 50%, 40% or 30% more preferably less than 20%, even more preferably less than 10%, less than 5%, less than 2% or even less than 1% 'leakage' of expressed (trans)gene product to other tissue or cells than endothelial tissue or cells.

The same applies *mutatis mutandis* for vascular endothelial cell-targeted, cardiac vascular endothelial cell-targeted, coronary artery endothelial cell-targeted expression, cardiac microvascular endothelial cell-targeted expression and liver sinusoidal endothelial cell-targeted expression, which may be considered as a particular form of endothelium-targeted expression. Throughout the application, where endothelium-targeted is mentioned in the context of expression, vascular endothelial cell-targeted, cardiac vascular endothelial cell-targeted, coronary artery endothelial cell-targeted expression, cardiac microvascular endothelial cell-targeted expression and liver sinusoidal endothelial cell-targeted expression are also explicitly envisaged.

The term "endothelial cell" as used herein encompasses all endothelial cell types, such as the cells forming a single cell layer that lines all blood vessels and regulates exchanges between the bloodstream and the surrounding tissues. Many endothelial cell types exist and their phenotypes vary between different organs, between different segments of the vascular loop within the same organ, and between neighboring endothelial cells of the same organ and blood vessel type. Non-limiting examples of such endothelial cells are: liver sinusoidal endothelial cells (LSEC); (micro)vascular endothelial cells from e.g. lung, heart (i.e. cardiac microvascular endothelial cells), intestine, skin, retina; arterial endothelial cells such as endothelial cells from coronary artery (CAECs), pulmonary artery, the aorta, umbilical artery and umbilical vein; extrahepatic endothelial cells from certain vascular beds; blood-brain barrier ECs; bone marrow ECs; and high endothelial venule cells (HEVs).

Sequences disclosed herein may be part of sequences of regulatory elements capable of controlling transcription of genes in endothelial cells, in particular in cardiac vascular endothelial cells, more particularly in coronary artery endothelial cells and cardiac microvascular endothelial cells, *in vivo,* in particular controlling the following genes: prothymosin alpha (PTMA) also known as TMSA; thymosin beta 10 (TMSB10) also known as TB10, PTMB10, THYB10 or MIG12; or Serpin Family E Member 1 (SERPINE1) also known as PAI, PLANH1 or PAIl.

Accordingly, in embodiments, the nucleic acid regulatory elements disclosed herein comprise a sequence from PTMA regulatory elements, i.e. regulatory elements that control expression of the PTMA gene *in vivo,* e.g. regulatory elements comprising SEQ ID NO:3 and functional fragments thereof as described herein elsewhere. In embodiments, the nucleic acid regulatory elements disclosed herein comprise a sequence from TMSB10 regulatory elements, i.e. regulatory elements that control expression of the TMSB10 gene *in vivo,* e.g. regulatory elements comprising SEQ ID NO:1 and functional fragments thereof as described herein elsewhere. In embodiments, the nucleic acid regulatory elements disclosed herein comprise a sequence from SERPINE1 regulatory elements, i.e. regulatory elements that control expression of the SERPINE1 gene *in vivo,* e.g. regulatory elements comprising SEQ ID NO:2 and functional fragments thereof as described herein elsewhere.

As used herein, the terms "sequence identity" and "identical sequence" and the like refer to the degree in sequence identity or similarity between two polymeric molecules, e.g., between two nucleic acid molecules, e.g., two DNA molecules. Sequence alignments and determination of sequence identity can be done, e.g., using the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250). Typically, the percentage sequence identity is calculated over the entire length of the sequence. As used herein, the term "substantially identical" denotes at least 90%, preferably at least 95%, such as 95%, 96%, 97%, 98% or 99%, sequence identity.

The term "functional fragment" as used in the application refers to fragments of the regulatory element sequences disclosed herein that retain the capability of regulating endothelium- or endothelial cell-targeted expression, i.e. they can still confer targeted expression in a specific tissue or cell, in particular in endothelium or an endothelial cell, and they are capable of regulating expression of a (trans)gene in the same way (although possibly not to the same extent) as the sequence from which they are derived. Functional fragments as defined herein preferably have at least 50%, at least 60%, at least 70%, at least 80%, at least 90%, at least 95% or about 100% of the regulatory capacities of the nucleic acid regulatory element sequence from which they are derived. Functional fragments may preferably comprise at least 20, at least 25, at least 30, at least 35, at least 40, at least 45, at least 50, at least 60, at least 70, at least 80, at least 90, at least 100, at least 120, at least 150, at least 200, at least 250, at least 300, at least 350, or at least 400 contiguous nucleotides from the sequence from which they are derived. Also preferably, functional fragments may comprise at least 1, more preferably at least 2, at least 3, or at least 4, even more preferably at least 5 or at least 10, yet more preferably all of the transcription factor binding sites (TFBS) that are present in the sequence from which they are derived.

In embodiments, the invention relates to a nucleic acid regulatory element for enhancing endothelium-targeted gene expression comprising, consisting essentially of, or consisting of a functional fragment of a sequence selected from the group consisting of: SEQ ID NO:3, SEQ ID NO:1, SEQ ID NO:2, and a sequence having at least 80%, preferably at least 85%, more preferably at least 90%, even more preferably at least 95%, such as 95%, 96%, 97%, 98%, or 99%, identity to (the full-length of) any one of these sequences, wherein said functional fragment comprises at least 20, preferably at least 25, more preferably at least 50, at least 100, at least 200 or at least 250, even more preferably at least 300, at least 350 or at least 400 contiguous nucleotides from the sequence from which it is derived. In embodiments, said functional fragments comprise at least 1, more preferably at least 2, at least 3, or at least 4, even more preferably at least 5 or at least 10, yet more preferably all of the transcription factor binding sites (TFBS) that are present in the sequence from which it is derived.

It is also possible to make nucleic acid regulatory elements that comprise an artificial sequence by combining two or more (e.g. two, three, four, five or more) identical or different nucleic acid regulatory element sequences disclosed herein or a functional fragment thereof as described herein.

Accordingly, in certain embodiments a nucleic acid regulatory element for enhancing endothelium-targeted gene expression is provided comprising, consisting essentially of or consisting of at least two sequences selected from the group consisting of: SEQ ID NO:3, SEQ ID NO:2, SEQ ID NO:1, and a sequence having at least 90%, preferably at least 95%, such as 95%, 96%, 97%, 98%, or 99%, identity to (the full-length of) any one of these sequences, or a functional fragment thereof. For example, disclosed herein is a nucleic acid regulatory element comprising, consisting essentially of, or consisting of 2, 3, 4, or 5 repeats of any one of SEQ ID NOs: 3, 1 and 2, or combinations thereof. The repeats can be combined in tandem or with one or more such as 1, 2, 3, 4, 5, 6, 7, 8, 9, 10 or more intervening or flanking nucleotides (e.g. nucleotides used for cloning purposes) between one or more of the repeats.

It is also possible to make nucleic acid regulatory elements that comprise an artificial sequence by combining one or more (e.g. one, two, three, four, five or more) nucleic acid regulatory element sequences disclosed herein or a functional fragment thereof as described herein with one or more endothelium-targeted regulatory elements which are known in the art. Non-limiting examples of known endothelium-targeted regulatory elements are disclosed in WO 2017/109039 A1 which is incorporated by reference in its entirety herein.

A particularly preferred example of an endothelium-targeted regulatory element for combining with a nucleic acid regulatory element disclosed herein is a nucleic acid regulatory element derived from the IFI27 gene promoter, more particularly a regulatory element comprising, consisting essentially of, or consisting of the sequence as defined in SEQ ID NO:17, a sequence having at least 85%, preferably at least 90%, more preferably at least 95%, such as 96%, 97%, 98% or 99%, identity to (the full-length of) the sequence as defined in SEQ ID NO:17, or a functional fragment thereof as described herein.

Accordingly, in certain embodiments a nucleic acid regulatory element for enhancing endothelium-targeted gene expression is provided comprising, consisting essentially of, or consisting of at least one, such as one or two or more, sequence selected from the group consisting of: SEQ ID NO:3, SEQ ID NO:2, SEQ ID NO:1, and a sequence having at least 90%, preferably at least 95%, such as 95%, 96%, 97%, 98%, or 99%, identity to (the full-length of) any one of these sequences, or a functional fragment thereof as described herein; and the sequence SEQ ID NO:17, a sequence having at least 85%, preferably at least 90%, more preferably at least 95%, such as 96%, 97%, 98% or 99%, identity to (the full-length of) SEQ ID NO: 17, or a functional fragment thereof as described herein. In particular embodiments, a nucleic acid regulatory element for enhancing endothelium-targeted gene expression is provided comprising, consisting essentially of, or consisting of the sequence set forth in SEQ ID NO:3 and the sequence set forth in SEQ ID NO:17. In particular embodiments, a nucleic acid regulatory element for enhancing endothelium-targeted gene expression is provided comprising, consisting essentially of, or consisting of the sequence set forth in SEQ ID NO:1 and the sequence set forth in SEQ ID NO:17. In particular embodiments, a nucleic acid regulatory element for enhancing endothelium-targeted gene expression is provided comprising, consisting essentially of, or consisting of the sequence set forth in SEQ ID NO:2 and the sequence set forth in SEQ ID NO:17.

The endothelium-targeted regulatory element(s) which are known in the art may be located upstream or downstream of the one or more (e.g. one, two, three, four, five or more) nucleic acid regulatory elements disclosed herein. Preferably, the one or more (e.g. one, two, three, four, five or more) nucleic acid regulatory elements disclosed herein are located upstream of the endothelium-targeted regulatory element(s) which are known in the art. The regulatory elements sequences can be combined in tandem or with one or more such as 1, 2, 3, 4, 5, 6, 7, 8, 9, 10 or more intervening or flanking nucleotides (e.g. nucleotides used for cloning purposes) between one or more of the regulatory elements sequences.

In case the regulatory element is provided as a single stranded nucleic acid, e.g. when using a single-stranded AAV vector, the complement strand is considered equivalent to the disclosed sequence. Hence, also disclosed herein is a nucleic acid regulatory element for enhancing endothelium-targeted gene expression comprising, consisting essentially of, or consisting of the complement of a sequence described herein, in particular a sequence selected from the group consisting of: SEQ ID NO:3, SEQ ID NO: 1, SEQ ID NO:2, and a sequence having at least 80%, preferably at least 85%, more preferably at least 90%, even more preferably at least 95%, such as 95%, 96%, 97%, 98%, or 99%, identity to (the full-length of) any one of these sequences, or a functional fragment thereof as described herein.

Also disclosed herein is a nucleic acid regulatory element for enhancing endothelium-targeted gene expression capable of hybridizing under stringent conditions to a nucleic acid regulatory element comprising, consisting essentially of, or consisting of a sequence selected from the group consisting of: SEQ ID NO:3, SEQ ID NO: 1, SEQ ID NO:2, and a sequence having at least 80%, preferably at least 85%, more preferably at least 90%, even more preferably at least 95%, such as 95%, 96%, 97%, 98%, or 99%, identity to (the full-length of) any one of these sequences, or a functional fragment thereof as described herein, or to its complement, preferably to a sequence selected from the group consisting of: SEQ ID NO:3, SEQ ID NO:1 and SEQ ID NO:2, or to its complement. Said nucleic acid regulatory elements do not need to be of equal length as the sequence they hybridize to. In preferred embodiments, the size of said hybridizing nucleic acid regulatory element does not differ more than 25% in length, in particular 20% in length, more in particular 15% in length, most in particular 10% in length from the sequence it hybridizes to.

The expression "hybridize under stringent conditions" refers to the ability of a nucleic acid molecule to hybridize to a target nucleic acid molecule (or the ability of a polynucleotide strand to anneal with a complementary strand through base pairing) under defined conditions of temperature and salt concentration. Specific hybridization is an indication that two nucleic acid sequences share a high degree of identity. Specific hybridization complexes form under permissive annealing conditions and remain hybridized after the "washing" step(s). Washing step(s) is/are particularly important in determining the stringency of a hybridization process, with more stringent conditions allowing less non-specific binding, i.e., binding between pairs of nucleic acid strands that are not perfectly matched. Permissive conditions for annealing of nucleic acid sequences are routinely determinable by one of ordinary skill in the art and may be consistent among hybridization experiments, whereas wash conditions may be varied among experiments to achieve the desired stringency, and therefore hybridization specificity.

In particular, the "high stringent conditions" as used herein may refer to the conditions under which nucleic acids having complementary strand sequences of base sequences shown in SEQ ID NOs:3, 1 or 2 can hybridize with nucleic acids having base sequences shown in SEQ ID NOs: 3, 1 or 2, showing complementarity of not less than about 70%, preferably not less than about 80%, more preferably not less than about 90%, particularly preferably not less than about 95%, 96%, 97%, 98% or 99% in the overlapping regions.

Typically, stringent hybridization conditions are no more than 25°C to 30°C (for example, 20°C, 15°C, 10°C or 5°C) below the melting temperature (Tm) of the native duplex. Methods of calculating Tm are well known in the art. By way of non-limiting example, representative salt and temperature conditions for achieving stringent hybridization may be: 1x SSC, 0.5% SDS at 65°C. The abbreviation SSC refers to a buffer used in nucleic acid hybridization solutions. One liter of the 20x (twenty times concentrate) stock SSC buffer solution (pH 7.0) contains 175.3 g sodium chloride and 88.2 g sodium citrate. A representative time period for achieving hybridization is 12 hours. Those skilled in this field can easily obtain desired stringency by suitably changing a salt concentration of the hybridization solution, a temperature of hybridization reaction, a mismatch number, a hybridization reaction time, a salt concentration of the washing solution, a washing temperature, and the like.

Preferably the regulatory elements as described herein are fully functional while being only of limited length. This allows their use in vectors or nucleic acid expression cassettes without unduly restricting their payload capacity. Accordingly, in embodiments, the nucleic acid regulatory element disclosed herein is a nucleic acid of 600 nucleotides or less, 550 nucleotides or less, 500 nucleotides or less, 450 nucleotides or less, or 425 nucleotides or less (i.e. the nucleic acid regulatory element has a maximal length of 600 nucleotides, 550 nucleotides, 500 nucleotides, 450 nucleotides or 425 nucleotides; or the nucleic acid regulatory element is maximal 600 nucleotides, 550 nucleotides, 500 nucleotides, 450 nucleotides or 425 nucleotides long; or a nucleic acid regulatory element of 600 nucleotides or less, 550 nucleotides or less, 500 nucleotides or less, 450 nucleotides or less, or 425 nucleotides or less).

However, it is to be understood that the disclosed nucleic acid regulatory elements retain regulatory activity (i.e. with regard to specificity and/or activity of transcription) and thus they particularly have a minimum length of 20 nucleotides, 25 nucleotides, 30 nucleotides, 35 nucleotides, 40 nucleotides, 45 nucleotides, 50 nucleotides, 100 nucleotides, 150 nucleotides, 200 nucleotides, 250 nucleotides, 300 nucleotides, 350 nucleotides or 400 nucleotides of the respective sequences of the regulatory elements disclosed herein.

The nucleic acid regulatory elements disclosed herein may be used in a nucleic acid expression cassette. Accordingly, in an aspect the invention provides for the use of the nucleic acid regulatory elements as described herein in a nucleic acid expression cassette.

In an aspect the invention provides a nucleic acid expression cassette comprising a nucleic acid regulatory element as described herein, operably linked to a promoter. In embodiments, the nucleic acid expression cassette does not contain a transgene. Such nucleic acid expression cassette may be used to drive expression of an endogenous gene.

In preferred embodiments, the nucleic acid expression cassette comprises a nucleic acid regulatory element as described herein, operably linked to a promoter and a transgene.

As used herein, the term 'nucleic acid expression cassette' refers to nucleic acid molecules that include one or more transcriptional control elements (such as, but not limited to promoters, enhancers and/or regulatory elements, polyadenylation sequences, and introns) that direct (trans)gene expression in one or more desired cell types, tissues or organs. Typically, they will also contain a transgene, although it is also envisaged that a nucleic acid expression cassette directs expression of an endogenous gene in a cell into which the nucleic acid cassette is inserted.

The term 'operably linked' as used herein refers to the arrangement of various nucleic acid molecule elements relative to each such that the elements are functionally connected and are able to interact with each other. Such elements may include, without limitation, a promoter, an enhancer and/or a regulatory element, a polyadenylation sequence, one or more introns and/or exons, and (a coding sequence of) a gene of interest to be expressed (i.e., the transgene), including transgenes expressing non-coding RNA as defined elsewhere herein. The nucleic acid sequence elements, when properly oriented or operably linked, act together to modulate the activity of one another, and ultimately may affect the level of expression of the transgene. By modulate is meant increasing, decreasing, or maintaining the level of activity of a particular element. The position of each element relative to other elements may be expressed in terms of the 5' terminus and the 3' terminus of each element, and the distance between any particular elements may be referenced by the number of intervening nucleotides, or base pairs, between the elements. As understood by the skilled person, operably linked implies functional activity, and is not necessarily related to a natural positional link. Indeed, when used in nucleic acid expression cassettes, the regulatory elements will typically be located immediately upstream of the promoter (although this is generally the case, it should definitely not be interpreted as a limitation or exclusion of positions within the nucleic acid expression cassette), but this needs not be the case *in vivo.* E.g., a regulatory element sequence naturally occurring downstream of a gene whose transcription it affects is able to function in the same way when located upstream of the promoter. Hence, according to a specific embodiment, the regulatory or enhancing effect of the regulatory element is position-independent.

In particular embodiments, the nucleic acid expression cassette comprises one nucleic acid regulatory element as described herein.

In alternative embodiments, the nucleic acid expression cassette comprises two or more, such as, e.g., 2, 3, 4, 5, 6, 7, 8, 9, or 10, nucleic acid regulatory elements as described herein, i.e. they are combined modularly to enhance their regulatory (and/or enhancing) effect. In further embodiments, at least two of the two or more nucleic acid regulatory elements are identical or substantially identical. In yet further embodiments, all of the two or more regulatory elements are identical or substantially identical. The copies of the identical or substantially identical nucleic acid regulatory elements may be provided as tandem repeats in the nucleic acid expression cassette, or they can be combined with one or more intervening or flanking nucleotides (e.g. nucleotides used for cloning purposes) between one or more of the regulatory elements. In alternative further embodiments, at least two of the two or more nucleic acid regulatory elements are different from each other, that is to say, are defined by a different sequence. The nucleic acid expression cassette may also comprise a combination of identical and substantially identical nucleic acid regulatory elements, and non-identical nucleic acid regulatory elements. The at least two nucleic acid regulatory elements can be combined in tandem or they can be combined with one or more intervening or flanking nucleotides (e.g. nucleotides used for cloning purposes) between one or more of the regulatory elements.

In yet alternative embodiments, the nucleic acid expression cassette comprises at least one nucleic acid regulatory element as described herein; and at least one endothelium-targeted nucleic acid regulatory element known in the art. The at least two nucleic acid regulatory elements can be combined in tandem or they can be combined with one or more intervening or flanking nucleotides (e.g. nucleotides used for cloning purposes) between one or more of the regulatory elements. In particular embodiments, the nucleic acid expression cassette comprises at least one nucleic acid regulatory element comprising, consisting essentially of or consisting of a sequence selected from the group consisting of: SEQ ID NO:3, SEQ ID NO:1, SEQ ID NO 2, and a sequence having at least 90%, preferably at least 95%, such as 95%, 96%, 97%, 98%, or 99%, identity to (the full-length of) any one of these sequences, or a functional fragment thereof as described herein; and a nucleic acid regulatory element comprising, consisting essentially of, or consisting of the sequence as defined in SEQ ID NO:17, a sequence having at least 85%, preferably at least 90%, more preferably at least 95%, such as 96%, 97%, 98% or 99%, identity to (the full-length of) SEQ ID NO: 17, or a functional fragment thereof as described herein. In further particular embodiments, the nucleic acid expression cassette comprises a nucleic acid regulatory element comprising, consisting essentially of, or consisting of the sequence set forth in SEQ ID NO:3; and a nucleic acid regulatory element comprising, consisting essentially of, or consisting of the sequence set forth in SEQ ID NO: 17. In further particular embodiments, the nucleic acid expression cassette comprises a nucleic acid regulatory element comprising, consisting essentially of, or consisting of the sequence set forth in SEQ ID NO:1; and a nucleic acid regulatory element comprising, consisting essentially of, or consisting of the sequence set forth in SEQ ID NO:17. In further particular embodiments, the nucleic acid expression cassette comprises a nucleic acid regulatory element comprising, consisting essentially of, or consisting of the sequence set forth in SEQ ID NO:2; and a nucleic acid regulatory element comprising, consisting essentially of, or consisting of the sequence set forth in SEQ ID NO:17.

As used in the application, the term 'promoter' refers to nucleic acid sequences that regulate, either directly or indirectly, the transcription of nucleic acid coding sequences to which they are operably linked (e.g. a transgene or endogenous gene). A promoter may function alone to regulate transcription or may act in concert with one or more other regulatory sequences (e.g. enhancers or silencers, or regulatory elements). In the context of the present application, a promoter is typically operably linked to a regulatory element as disclosed herein to regulate transcription of a (trans)gene. When a regulatory element as described herein is operably linked to both a promoter and a transgene, the regulatory element can (1) confer a significant degree of endothelium-targeted or endothelial cell-targeted expression *in vivo* (and/or in endothelial cells or tissues, or cell lines derived from endothelial cells or tissue *in vitro*) of the transgene, and/or (2) can increase the level of expression of the transgene in endothelial cells or tissue *in vivo* (and/or in endothelial cells or tissue, or cell lines derived from enodothelial cells or tissue *in vitro*).

The promoter may be homologous (i.e. from the same species as the animal, in particular mammal, to be transfected with the nucleic acid expression cassette) or heterologous (i.e. from a source other than the species of the animal, in particular mammal, to be transfected with the expression cassette). As such, the source of the promoter may be any virus, any unicellular prokaryotic or eukaryotic organism, any vertebrate or invertebrate organism, or any plant, or may even be a synthetic promoter (i.e. having a non-naturally occurring sequence), provided that the promoter is functional in combination with the regulatory elements described herein. In embodiments, the promoter is a mammalian promoter, in particular a murine or human promoter.

Furthermore, the promoter does not need to be the promoter of the transgene in the nucleic acid expression cassette, although it is possible that the transgene is transcribed from its own promoter.

In embodiments, the promoter may be heterologous to the regulatory element, i.e. the promoter does not need to be the promoter of the regulatory element in the nucleic acid expression cassette.

The promoter may be an inducible or constitutive promoter.

The nucleic acid regulatory elements disclosed herein in principle direct endothelium-targeted gene expression even from a promoter that itself is not endothelium-targeting. Hence, the regulatory elements disclosed herein can be used in nucleic acid expression cassettes in conjunction with any promoter, in particular the promoter may either be tissue-specific, in particular an endothelium-targeted promoter, or ubiquitously expressed.

Non-limiting examples of ubiquitously expressed promoters include the cytomegalovirus (CMV) promoter, the CAG promoter (a synthetic promoter comprising the cytomegalovirus (CMV) early enhancer element fused to the promoter, the first exon and the first intron of chicken beta-actin gene and the splice acceptor of the rabbit beta-globin gene), RNA polymerase II (pol II) promoters, RNA polymerase III (pol III) promoters (e.g. an U6 pol III promoter, in particular the human U6 small nuclear promoter (U6), or a HI pol III promoter, in particular the human HI promoter (HI)) and chimeric pol III promoters.

In embodiments, the nucleic acid expression cassettes disclosed herein comprise an endothelium-targeted promoter. An endothelium-targeted promoter as used herein refers to a promoter which preferentially or predominantly expresses a (trans)gene in endothelial cells or tissue and/or expression of the (trans)gene in other (i.e. non-endothelial) cells and tissues is minimal. The use of an endothelial-targeted promoter, may increase targeted expression in endothelial cells or tissue, and/or avoid or reduce leakage of (trans)gene expression in other tissues or cells. Non-limiting examples of endothelium-targeted promoters include an Intercellular Adhesion Molecule 2 (ICAM2) promoter, a coagulation factor VIII (FVIII) promoter, an endothelin-1 (EDN1) promoter, a cadherin-5 (CDH5) or VE-cadherin promoter (e.g. as described in Prandini et al. 2005 Oncogene 24:2992-3001), an endoglin (ENG) promoter, an Interferon Alpha Inducible Protein 27 (IFI27) promoter, a von Willebrand factor (VWF) promoter, an Endothelial cell-specific chemotaxis regulator (ECSCR) promoter, a Platelet And Endothelial Cell Adhesion Molecule 1 (PECAM1) promoter, a Hedgehog Interacting Protein (HHIP) promoter, a Tyrosine Kinase With Immunoglobulin Like And EGF Like Domains 1 (TIE1) promoter, a Hyaluronidase 2 (HYAL2) promoter, and an Fms-Related Tyrosine Kinase 1 (FLT1) promoter.

In particular embodiments, the promoter is an endothelium-targeted promoter selected from the group consisting of: an ICAM2 promoter, a FVIII promoter, and an EDN1 promoter, preferably an ICAM2 promoter.

In particular embodiments, the promoter is the ICAM2 promoter as defined by SEQ ID NO:18

(GTCTCCCAGGCATGACTCCAACAATGCATCCCATGGGATTTGGGGTTCCCCAGATCTGGGGCTTGTAG
GCCTGACTCTCCCCTGTGCACACGTCTCATACACGCATGCGTGCACCCATTGCCTGCCCCGCCCCTTGC
ACAGGGAGTCAGCAGGGAGGACTGGGTTATGCCCTGCTTATCAGCAGCTTCCCAGCTTCCTCTGCCTGG

ATTCTTAGAGGCCTGGGGTCCTAGAACGAGCTGGTGCACGTGGCTTCCCAAAGATCTCTCAGATAATGA
GAGGAAATGCAGTCATCAGTTTGCAGAAGGCTAGGGATTCTGGGCCATAGCTCAGACCTGCGCCCACCA
TCTCCCTCCAGGCAGCCCTTGGCTGGTCCCTGCGAGCCCGTGGAGACTGCCAGTC).

In particular embodiments, the promoter is the FVIII promoter as defined by SEQ ID NO:19

(GAGCTCACCATGGCTACATTCTGATGTAAAGAGATATATCCTATACCTGGGCCAAATGTAAACAGCCT
GGAAAAGTGTTAGGTTAAAAACAAAACAAAATAAATAAATGAATAAATGCCAGGTGGTTATGAGTGCTA
TTGAGAAAAATGAAGCCAAGAGGGATATCAGTGATGCAGGTGGGGGTAAAGAGCTTACAACATAAATGT
GGTGTTCCATATTTAAACCTCATTCAACAGGGAAGATTGGAGCTGAAATGTGAAGGAGTTGTGGGAGTG
GAACTACGTGGAAATCTGGGGGAAAGGTGTTTTGGGTAAAAGAAATAGCAAGTGTTGAGGTCCAGGGGC
ATGAGTGTGCTTGATATTTTAGGGAAGAGTAAGGAGACCAGTATAACCAGAGTGAGATGAGACTACAGA
GGTCAGGAGAAAGGGCATGCAGACCATGTGGGATGCTCTAGGACCTAGGCCATGGTAAAGATGTAGGGT
TTTACCCTGATGGAGGTCAGAAGCCATTGGAGGATTCTGAGAAGAGGAGTGACAGGACTCGCTTTATAG
TTTTAAATTATAACTATAAATTATAGTTTTTAAAACAATAGTTGCCTAACCTCATGTTATATGTAAAAC
TACAGTTTTAAAAACTATAAATTCCTCATACTGGCAGCAGTGTGAGGGGCAAGGGCAAAAGCAGAGAGA
CTAACAGGTTGCTGGTTACTCTTGCTAGTGCAAGTGAATTCTAGAATCTTCGACAACATCCAGAACTTC
TCTTGCTGCTGCCACTCAGGAAGAGGGTTGGAGTAGGCTAGGAATAGGAGCACAAATTAAAGCTCCTGT
TCACTTTGACTTCTCCATCCCTCTCCTCCTTTCCTTAAAGGTTCTGATTAAAGCAGACTTATGCCCCTA
CTGCTCTCAGAAGTGAATGGGTTAAGTTTAGCAGCCTCCCTTTTGCTACTTCAGTTCTTCCTGTGGCTG
CTTCCCACTGATAAAAAGGAAGCAATCCTATCGGTTACTGCTTAGTGCTGAGCACATCCAGTGGGTAAA
GTTCCTTAAAATGCTCTGCAAAGAAATTGGGACTTTTCATTAAATCAGAAATTTTACTTTTTTCCCCTC
CTGGGAGCTAAAGATATTTTAGAGAAGAATTAACCTTTTGCTTCTCCAGTTGAACATTTGTAGCAATAA
GTC)

In particular embodiments, the promoter is the EDN1 promoter as defined by SEEQ ID NO:20

(CTTGTGCGGGGAATAAATAGAATATTCTGAGGTGCATTCCTCCTTCCTATGTTAGGCAACATTCCTTG
ACCCTCCTCGGCCCCCAAGCCAGGTTGCGTTTTTTTCTGCCATTTAGAAGGGTTTTCCTTTTTGTCCTA
GTAAAACATCAGCCCCTGTAGCTCTTCATCTCCCCCTGGTGTTCTTCTCCCGCCATGTCTTAAGATTGG
TGGCACCGACCAATCTTAAGATTTAAGTTCTGTGTGAAAAACACCTTTGCTTTTCAATCAGTTTATCAG
CCTCCTCCGCAGGGGAAGTGTGGACACACAAAAGAACTTATCGGGGCTTCTCATCAGTGATAGGGAAAA
GACTGGGCATGTGCCTAAACGAGCTCTGATGTTATTTTTAAGCTCCCTTTCTTGCCAATCCCTCACGGA
TCTTTCTCCGATAGATGCAAAGAACTTCAGCAAAAAAGACCCGCAGGAAGGGGCTTGAAGAGAAAAGTA
CGTTGATCTGCCAAAATAGTCTGACCCCCAGTAGTGGGGCAGTGACGAGGGAGAGCATTCCCTTGTTTG
ACTGAGACTAGAATCGGAGAGACATAAAAGGAAAATGAAGCGAGCAACAATTAAAAAAAATTCCCCGCA
CACAACAATACAATCTATTTAAACTGTGGCTCATACTTTTCATACCAATGGTATGACTTTTTTTCTGGA
GTCCCCTCTTCTGATTCTTGAACTCCGGGGCTGGCAGCTTGCAAAGGGGAAGCGGACTCCAGCACTGCA
CGGGCAGGTTTAGCAAAGGTCTCTAATGGGTATTTTCTTTTTCTTAGCCCTGCCCCCGAATTGTCAGAC
GGCGGGCGTCTGCCTCTGAAGTTAGCAGTGATTTCCTTTCGGGCCTGGCCTTATCTCCGGCTGCACGTT
GCCTGTTGGTGACTAATAACACAATAACATTGTCTGGGGCTGGAATAAAGTCGGAGCTGTTTACCCCCA
CTCTAATAGGGGTTCAATATAAAAAGCCGGCAGAGAGCTGTCCAAGTCAGACGCGCCTCTGCATCTGCG

CCAGGCGAACGGGTCCTGCGCCTCCTGCAGTCCCAGCTCTCCACCGCCGCGTGCGCCTGCAGACGCTCC
GCTCGCTGCCTTCTCTCCTGGCAGGCGCTGCCTTTTCTCCCCGTTAAAAGGGCACTTGGGCTGAAGGAT
CGCTTTGAGATCTGAGGAACCCGCAGCGCTTTGAGGGACCTGAAGCTGTTTTTCTTCGTTTTCCTTTGG
GTTCAGTTTGAACGGGAGGTTTTTGATCCCTTTTTTTCAGA)

To minimize the length of the nucleic acid expression cassette, the regulatory elements may be linked to minimal promoters, or shortened versions of the promoters described herein. A 'minimal promoter' (also referred to as basal promoter or core promoter) as used herein is part of a full-size promoter still capable of driving expression, but lacking at least part of the sequence that contributes to regulating (e.g. tissue-targeted) expression. This definition covers both promoters from which (tissue-targeted) regulatory elements have been deleted- that are capable of driving expression of a gene but have lost their ability to express that gene in a tissue-targeted fashion and promoters from which (tissue-targeted) regulatory elements have been deleted that are capable of driving (possibly decreased) expression of a gene but have not necessarily lost their ability to express that gene in a tissue-targeted fashion. Preferably, the promoter contained in the nucleic acid expression cassette disclosed herein is 1500 nucleotides or less in length, 1000 nucleotides or less in length, 900 nucleotides or less, 800 nucleotides or less, 700 nucleotides or less, 600 nucleotides or less, 500 nucleotides or less, 400 nucleotides or less, 300 nucleotides or less, or 250 nucleotides or less.

The term 'transgene' as used herein refers to particular nucleic acid sequences encoding a polypeptide or a portion of a polypeptide to be expressed in a cell into which the nucleic acid sequence is introduced. However, it is also possible that transgenes are expressed as non-coding RNA, e.g. to control (e.g. lower) the amount of a particular polypeptide in a cell into which the nucleic acid sequence is inserted.

How the nucleic acid sequence is introduced into a cell is not essential to the invention, it may for instance be through integration in the genome or as an episomal plasmid. Of note, expression of the transgene may be restricted to a subset of the cells into which the nucleic acid sequence is introduced. The term 'transgene' is meant to include (1) a nucleic acid sequence that is not naturally found in the cell (i.e., a heterologous nucleic acid sequence); (2) a nucleic acid sequence that is a mutant form of a nucleic acid sequence naturally found in the cell into which it has been introduced; (3) a nucleic acid sequence that serves to add additional copies of the same (i.e., homologous) or a similar nucleic acid sequence naturally occurring in the cell into which it has been introduced ; or (4) a silent naturally occurring or homologous nucleic acid sequence whose expression is induced in the cell into which it has been introduced.

The transgene may be homologous or heterologous to the promoter (and/or to the animal, in particular mammal, in which it is introduced, e.g. in cases where the nucleic acid expression cassette is used for gene therapy).

The transgene may be a full length cDNA or genomic DNA sequence, or any fragment, subunit or mutant thereof that has at least some biological activity. In particular, the transgene may be a minigene, i.e. a gene sequence lacking part, most or all of its intronic sequences. The transgene thus optionally may contain intron sequences. Optionally, the transgene may be a hybrid nucleic acid sequence, i.e., one constructed from homologous and/or heterologous cDNA and/or genomic DNA fragments. By 'mutant form' is meant a nucleic acid sequence that contains one or more nucleotides that are different from the wild-type or naturally occurring sequence, i.e., the mutant nucleic acid sequence contains one or more nucleotide substitutions, deletions, and/or insertions. The nucleotide substitution, deletion, and/or insertion can give rise to a gene product (i.e. e., protein or nucleic acid) that is different in its amino acid/nucleic acid sequence from the wild type amino acid/nucleic acid sequence. Preparation of such mutants is well known in the art. In some cases, the transgene may also include a sequence encoding a leader peptide or signal sequence such that the transgene product will be secreted from the cell.

In particular embodiments, the transgene is codon-optimized. As used herein the terms "codonoptimization", "codon-optimized" and the like refer to changes in the codon composition of a nucleic acid sequence, in particular a transgene, without altering the amino acid sequence, e.g. for optimal expression in a host cell or organism.

The size of the transgenes contained in the nucleic acid expression cassettes and vectors described herein is not particularly limited, but may be determined by the packaging capacity of the vector as readily known to the skilled person. In embodiments, e.g. when using AAV vectors, the transgene contained in the nucleic acid expression cassette disclosed herein is less than 5 kb, such as less than 4 kb, less than 3 kb or less than 2 kb, in length. In embodiments, e.g. when using adenoviral vectors, the transgene contained in the nucleic acid expression cassette disclosed herein is less than 36 kb, such as less than 35 kb, less than 30 kb, less than 25 kb, less than 20 kb or less than 15 kb, in length. In embodiments, e.g. when using lentiviral vectors, the transgene contained in the nucleic acid expression cassette disclosed herein is less than 10 kb, such as less than 8 kb, less than 6 kb, less than 5 kb or less than 4 kb, in length.

The transgene that may be contained in the nucleic acid expression cassettes described herein typically encodes a transgene product such as RNA or a polypeptide (protein).

In embodiments, the transgene encodes a non-coding RNA (ncRNA). As used herein, "non-coding RNA" refers to an RNA molecule that is transcribed from DNA but not translated into a protein. These non-coding RNA molecules include but are not limited to molecules that exert their function through RNA interference (e.g. short hairpin RNA (shRNA), small interfering RNA (siRNA)), microRNA regulation (e.g. microRNA (miRNA), which can be used to control expression of specific genes), long non-coding RNA (lncRNA), circular RNA (cRNA), catalytic RNA, antisense RNA, enhancer RNA (eRNA), RNA aptamers, guide RNA used in the context of CRISPR systems, etc.

In embodiments, the transgene encodes a therapeutic protein or an immunogenic protein.

In embodiments, the transgene encodes a therapeutic protein. The therapeutic protein may be a secretable protein. Secretable proteins, in particular secretable therapeutic proteins, may include, without limitation, clotting factors, growth factors, antibodies (e.g. antibodies directed against a clotting factor or a growth factor, or their cognate receptors), cytokines, chemokines, matrix metalloproteinases, tissue inhibitors of metalloproteinases (TIMPs), etc. Non-limiting examples of therapeutic proteins, in particular secretable therapeutic proteins, include coagulation factor VIII (FVIII), coagulation factor VII (FVII), coagulation factor IX (FIX), coagulation factor XI (FXI), tissue factor (TF), von Willebrand factor (vWF), tissue factor pathway inhibitor (TFPI), a disintegrin and metalloproteinase with a thrombospondin type 1 motif, member 13 (ADAMTS13), hepatocyte growth factor (HGF), vascular endothelial growth factor (VEGF), placental growth factor (PLGF), fibroblast growth factor (FGF), anti-VEGF antibody, anti-PLGF antibody, soluble fms-like tyrosine kinase-1 (sFLT1), α1-antitrypsin (AAT), apolipoprotein A-I (apoA-I), tissue inhibitor of metalloproteinase-3 (TIMP-3), erythropoietin, lipoprotein lipase, adiponectin or insulin.

The therapeutic protein may also be a non-secreted protein. Non-limiting examples of non-secreted proteins, in particular non-secreted therapeutic proteins, include structural proteins, e.g. proteins modulating vascular relaxation and vasoconstriction, vascular inflammation, neointima formation, atherosclerosis, angiogenesis and vasculogenesis; enzymes, e.g. nitric oxide synthase (NOS); and transcription factors, e.g. hypoxia-inducible factor 1-alpha (HIF-1α ).

The transgene may also encode a guide RNA or an endonuclease (e.g. Cas9).

In embodiments, the transgene encodes an immunogenic protein. Non-limiting examples of immunogenic proteins include epitopes and antigens derived from a pathogen (e.g. SARS-CoV-2 antigens, dengue virus antigens, etc.).

As used herein, the term "immunogenic" refers to a substance or composition capable of eliciting an immune response.

In certain embodiments, the transgene encodes a FVIII. The term "coagulation factor VIII" has the meaning as known in the art. Synonyms of coagulation factor VIII are "FVIII" or "anti-hemophilic factor" or "AHF" and can be used interchangeably herein. The term "coagulation factor VIII" encompasses, for example, the human protein having the amino acid sequence as defined in Uniprot accession number P00451. Preferably, said FVIII is a FVIII wherein the B domain is deleted (i.e. B domain deleted FVIII, also referred to as BDD FVIII or FVIIIΔB herein). The term "B domain deleted FVIII" encompasses for example, but without limitation, FVIII mutants wherein whole or a part of the B domain is deleted and FVIII mutants wherein the B domain is replaced by a linker. The term also encompasses FVIII variants comprising one or more modifications in the B domain sequences, FVIII variants showing improved secretion, and chimeric FVIII.

Other sequences may be incorporated in the nucleic acid expression cassette disclosed herein as well, typically to further increase or stabilize the expression of the transgene product (e.g. introns and/or polyadenylation sequences).

Any intron can be utilized in the expression cassettes described herein. The term "intron" encompasses any portion of a whole intron that is large enough to be recognized and spliced by the nuclear splicing apparatus. Typically, short, functional, intron sequences are preferred in order to keep the size of the expression cassette as small as possible which facilitates the construction and manipulation of the expression cassette. In some embodiments, the intron is obtained from a gene that encodes the protein that is encoded by the coding sequence within the expression cassette. The intron can be located 5' to the coding sequence, 3' to the coding sequence, or within the coding sequence. An advantage of locating the intron 5' to the coding sequence is to minimize the chance of the intron interfering with the function of the polyadenylation signal. In embodiments, the nucleic acid expression cassette disclosed herein further comprises an intron. Non-limiting examples of suitable introns are Minute Virus of Mice (MVM) intron, beta-globin intron (betaIVS-II), factor IX (FIX) intron A, Simian virus 40 (SV40) small-t intron, and beta-actin intron. Preferably, the intron is a minute virus of mouse (MVM) intron, more preferably the MVM intron as defined by SEQ ID NO:21

(AAGAGGTAAGGGTTTAAGGGATGGTTGGTTGGTGGGGTATTAATGTTTAATTACCTGGAGCACCTGCC

TGAAATCACTTTTTTTCAGGTTGG).

Any polyadenylation signal (also referred to herein as "polyadenylation site", "poly A", or "pA") that directs the synthesis of a polyA tail is useful in the expression cassettes described herein, examples of those are well known to one of skill in the art. Exemplary polyadenylation signals include, but are not limited to, polyA sequences derived from the Simian virus 40 (SV40) late gene, the bovine growth hormone (BGH) polyadenylation signal, the minimal rabbit β-globin (mRBG) gene, and synthetic poly A sites (SPA or synt.pA), such as the synthetic pA site as described in Levitt et al. (1989, Genes Dev 3:1019-1025).

Preferably, the polyadenylation signal is a synthetic polyadenylation signal, more preferably the polyadenylation signal defined by SEQ ID NO:22
(AATAAAAGATCTTTATTTTCATTAGATCTGTGTGTTGGTTTTTTGTGTG).

In particular embodiments, the invention provides a nucleic acid expression cassette comprising a nucleic acid regulatory element comprising or consisting of a sequence selected from the group consisting of: SEQ ID NO:3, 1, 2, and a sequence having at least 95% identity to (the full length of) any one of said sequences, operably linked to a promoter, preferably an endothelium-targeted promoter, more preferably an ICAM2 promoter (preferably the ICAM2 promoter as defined by SEQ ID NO:18), and a transgene.

In particular embodiments, the invention provides a nucleic acid expression cassette comprising a nucleic acid regulatory element comprising or consisting of a sequence selected from the group consisting of: SEQ ID NO:3, 1, 2, and a sequence having at least 95% identity to (the full length of) any one of said sequences, and a nucleic acid regulatory element comprising or consisting of the sequence set forth in SEQ ID NO:17, or a sequence having at least 95% identity to (the full length of) SEQ ID NO:17, wherein said nucleic acid regulatory elements are operably linked to a promoter, preferably an endothelium-targeted promoter, more preferably an ICAM2 promoter (preferably the ICAM2 promoter as defined by SEQ ID NO:18), and a transgene.

In further embodiments, the nucleic acid expression cassette further comprises an MVM intron, preferably the MVM intron defined by SEQ ID NO:21. In yet further embodiments, the nucleic acid expression cassette further comprises a polyadenylation signal, preferably a synthetic polyadenylation signal, more preferably the polyadenylation signal defined by SEQ ID NO:22.

The nucleic acid regulatory element and the nucleic acid expression cassette disclosed herein may be used as such, or typically, they may be part of a nucleic acid vector. Accordingly, a further aspect relates to the use of a nucleic acid regulatory element as described herein or a nucleic acid expression cassette as described herein in a vector, in particular a nucleic acid vector.

In an aspect, the invention also provides a vector comprising a nucleic acid regulatory element as disclosed herein. In further embodiments, the vector comprises a nucleic acid expression cassette as disclosed herein.

The term 'vector' as used in the application refers to nucleic acid molecules, e.g. double-stranded DNA, which may have inserted into it another nucleic acid molecule (the insert nucleic acid molecule) such as, but not limited to, a cDNA molecule. The vector is used to transport the insert nucleic acid molecule into a suitable host cell. A vector may contain the necessary elements that permit transcribing the insert nucleic acid molecule, and, optionally, translating the transcript into a polypeptide. The insert nucleic acid molecule may be derived from the host cell, or may be derived from a different cell or organism. Once in the host cell, the vector can replicate independently of, or coincidental with, the host chromosomal DNA, and several copies of the vector and its inserted nucleic acid molecule may be generated. The vectors can be episomal vectors (i.e., that do not integrate into the genome of a host cell), or can be vectors that integrate into the host cell genome. The term 'vector' may thus also be defined as a gene delivery vehicle that facilitates gene transfer into a target cell. This definition includes both non-viral and viral vectors.Non-viral vectors include but are not limited to cationic lipids, liposomes, nanoparticles, PEG, PEI, plasmid vectors (e.g. pUC vectors, bluescript vectors (pBS) and pBR322 or derivatives thereof that are devoid of bacterial sequences (minicircles)) transposons-based vectors (e.g. PiggyBac (PB) vectors or Sleeping Beauty (SB) vectors), etc. Viral vectors are derived from viruses and include but are not limited to retroviral, lentiviral, adeno-associated viral, adenoviral, herpes viral, hepatitis viral vectors or the like. Typically, but not necessarily, viral vectors are replication-deficient as they have lost the ability to propagate in a given cell since viral genes essential for replication have been eliminated from the viral vector. However, some viral vectors can also be adapted to replicate specifically in a given cell, such as e.g. a cancer cell, and are typically used to trigger the (cancer) cell-specific (onco)lysis. Virosomes are a non-limiting example of a vector that comprises both viral and non-viral elements, in particular they combine liposomes with an inactivated HIV or influenza virus (Yamada et al., 2003). Another example encompasses viral vectors mixed with cationic lipids.

In preferred embodiments, the vector is a viral vector, such as a retroviral, lentiviral (LV), adenoviral (AV), or adeno-associated viral (AAV) vector, preferably an adeno-associated viral (AAV) vector, a lentiviral (LV) vector or an adenoviral (AV) vector, more preferably an AAV vector.

AAV vectors can be used as self-complementary, double-stranded AAV vectors (scAAV) in order to overcome one of the limiting steps in AAV transduction (i.e. single-stranded to double-stranded AAV conversion) (McCarty, 2001, 2003; Nathwani et al, 2002, 2006, 2011; Wu et al., 2008). Also the use of single-stranded AAV vectors (ssAAV) is encompassed herein. In particular embodiments, the vector is a single-stranded AAV (ssAAV) vector.

Different serotypes of AAVs have been isolated and characterized, such as, for example AAV serotype 2, AAV serotype 5, AAV serotype 8, and AAV serotype 9, and all AAV serotypes are contemplated herein. The selection of an AAV serotype may be determined by the envisaged application and/or the host organism. In particular embodiments, the vector is an AAV9 vector. Capsids can be derived from a different serotype or can be specifically modified to enhance endothelial cell transduction either by evolution or selection, antibody (nanobody) engineering or the use of DARPin (Work et al. (2006) Mol Ther. 13:683-93; Münch et al. (2015) Nat Commun. 6:6246; Buchholz et al. (2015) Trends Biotechnol. 33:777-90; White et al. (2004) Circulation. 109:513-519).

Production of AAV vector particles can be achieved e.g. by transient co-transfection of an AVV vector and an AAV helper construct encoding AAV capsids in suspension-adapted mammalian HEK293 cells, as described (Chahal et al. Journal of Virological Methods. 196: 163-173 (2014); Grieger et al., Molecular Therapy. 24: 287-297 (2016); Blessing et al., Molecular Therapy Methods & Clinical Development. 13: 14-26 (2019)), or by infection of Spodoptera frugiperda (Sf9) insect cells using the baculovirus expression vector system (BEVS), as described (Kotin et al. Human Gene Therapy. 28: 350-360 (2017)), followed by a purification step. Purification may be based on cesium chloride (CsCl) density gradient ultracentrifugation, as described (VandenDriessche et al., 2007), or using chromatographic techniques or columns or by immunoaffinity as known in the art.

In other embodiments, the vector is a non-viral vector, preferably a plasmid, a minicircle, or a transposon-based vector, such as a Sleeping Beauty (SB)-based vector or piggyBac (PB)-based vector.

For gene therapy, the transposon-based vectors may be administered in combination with a vector or mRNA encoding a transposase or the transposase. A suitable SB transposon has been described in Ivies et al. (1997 Cell. 91:501-510) and its hyperactive and/or codon-optimized versions, including SB100X, as described in Mates et al. (2009 Nat Genet. 41, 753-6). Alternatively, PiggyBac-based transposons can be used. For example, the PiggyBac-derived transposon-based vector can be administered with wild-type PiggyBac transposase (Pbase) or mouse codon-optimized PiggyBac transposase (mPBase). Preferably, said transposases are hyperactive transposases, such as, for example, hyperactive PB (hyPB) transposase containing seven amino acid substitutions (I30V, S103P, G165S, M282V, S509G, N538K, N570S) as described in Yusa et al. (2011 Proc Natl Acad Sci USA. 108:1531-6 ), which is specifically incorporated by reference herein. Other transposase modifications may include the use of targeting domains or hybrid systems such as CRISPR-based transposase targeting (Kovak et al. Elife. 2020;9:e53868).

Transposon and/or transposase constructs can be delivered by hydrodynamic injection or using non-viral transfection reagents such as lipid nanoparticles (LNPs), nanocarriers or exosomes to transfect endothelial cells.

In yet other embodiments, the vector comprises viral and non-viral elements.

The nucleic acid expression cassettes and vectors disclosed herein may be used, for example, to express proteins that are normally expressed and utilized in endothelial cells (e.g. structural proteins, enzymes, transcription factors), or to express proteins that are expressed in endothelial cells and that are then exported to the blood stream for transport to other portions of the body (e.g. secretable proteins). For example, the expression cassettes and vectors disclosed herein may be used to express a therapeutic amount of a gene product (such as a polypeptide, in particular a therapeutic protein, or RNA) for therapeutic purposes, in particular for gene therapy. Typically, the gene product is encoded by the transgene within the expression cassette or vector, although in principle it is also possible to increase expression of an endogenous gene for therapeutic purposes. In an alternative example, the expression cassettes and vectors disclosed herein may be used to express an immunological amount of a gene product (such as a polypeptide, in particular an immunogenic protein, or RNA) for vaccination purposes.

The nucleic acid expression cassettes and vectors as taught herein may be formulated in a pharmaceutical composition with a pharmaceutically acceptable excipient, i.e., one or more pharmaceutically acceptable carrier substances and/or additives, e.g., buffers, carriers, excipients, stabilisers, etc. The pharmaceutical composition may be provided in the form of a kit.

The term "pharmaceutically acceptable" as used herein is consistent with the art and means compatible with the other ingredients of the pharmaceutical composition and not deleterious to the recipient thereof.

Accordingly, a further aspect of the invention relates to a pharmaceutical composition comprising a nucleic acid expression cassette or a vector described herein.

The use of nucleic acid regulatory elements described herein for the manufacture of these pharmaceutical compositions is also disclosed herein.

In a further aspect, the invention relates to the nucleic acid regulatory elements, the nucleic acid expression cassettes, the vectors, or the pharmaceutical compositions described herein for use in medicine.

As used herein, the terms "treat" or "treatment" refer to both therapeutic treatment and prophylactic or preventative measures. Beneficial or desired clinical results include, but are not limited to, prevention of an undesired clinical state or disorder, reducing the incidence of a disorder, alleviation of symptoms associated with a disorder, diminishment of extent of a disorder, stabilized (i.e., not worsening) state of a disorder, delay or slowing of progression of a disorder, amelioration or palliation of the state of a disorder, remission (whether partial or total), whether detectable or undetectable, or combinations thereof.

"Treatment" can also mean prolonging survival as compared to expected survival if not receiving treatment.

As used herein, the terms "therapeutic treatment" or "therapy" and the like, refer to treatments wherein the object is to bring a subjects body or an element thereof from an undesired physiological change or disorder to a desired state, such as a less severe or unpleasant state (e.g., amelioration or palliation), or back to its normal, healthy state (e.g., restoring the health, the physical integrity and the physical wellbeing of a subject), to keep it at said undesired physiological change or disorder (e.g., stabilization, or not worsening), or to prevent or slow down progression to a more severe or worse state compared to said undesired physiological change or disorder.

As used herein the terms "prevention", "preventive treatment" or "prophylactic treatment" and the like encompass preventing the onset of a disease or disorder, including reducing the severity of a disease or disorder or symptoms associated therewith prior to affliction with said disease or disorder. Such prevention or reduction prior to affliction refers to administration of the nucleic acid regulatory elements, the nucleic acid expression cassettes, the vectors, or the pharmaceutical compositions described herein to a patient that is not at the time of administration afflicted with clear symptoms of the disease or disorder. "Preventing" also encompasses preventing the recurrence or relapse-prevention of a disease or disorder for instance after a period of improvement.

A further aspect relates to the nucleic acid regulatory elements, the nucleic acid expression cassettes, the vectors, or the pharmaceutical compositions described herein may be for use in gene therapy, in particular endothelium-directed gene therapy.

Also disclosed herein is the use of the nucleic acid regulatory elements, the nucleic acid expression cassettes, the vectors, or the pharmaceutical compositions described herein for the manufacture of a medicament for gene therapy, in particular endothelium-directed gene therapy.

Also disclosed herein is a method for gene therapy, in particular endothelium-directed gene therapy, in a subject in need of said gene therapy comprising:
- introducing in the subject, in particular in an endothelial cell of the subject, a nucleic acid expression cassette, a vector or a pharmaceutical composition described herein, wherein the nucleic acid expression cassette, the vector or the pharmaceutical composition comprises a nucleic acid regulatory element described herein, operably linked to a promoter and a transgene; and
- expressing a therapeutically effective amount of the transgene product in the subject, in particular in the endothelial cell of the subject.

In particular embodiments, the nucleic acid expression cassette or the vector is introduced into a liver sinusoidal endothelial cell, for example, but without limitation, for liver-directed gene therapy. In particular embodiments, the nucleic acid expression cassette or the vector is introduced into a cardiac endothelial cell, e.g. for heart-directed gene therapy.

The transgene product may be a polypeptide, in particular a therapeutic protein, or a non-coding RNA (ncRNA) molecule. Non-limiting examples of therapeutic proteins and ncRNA molecules have been disclosed above in connection with the transgene. The transgene product may also be RNA, such as a guideRNA, or a nuclease such as a zinc finger nuclease (ZFN), a Transcription Activator-Like Effector Nucleases (TALEN), Cas9 for DNA or RNA editing.

Gene therapy protocols have been extensively described in the art. These include, but are not limited to, intramuscular injection of plasmid (naked or in liposomes), hydrodynamic gene delivery in various tissues, including muscle, interstitial injection, instillation in airways, application to endothelium, and intravenous or intra-arterial administration. Various devices have been developed for enhancing the availability of DNA to the target cell. A simple approach is to contact the target cell physically with catheters or implantable materials containing DNA. Another approach is to utilize needle-free, jet injection devices which project a column of liquid directly into the target tissue under high pressure. These delivery paradigms can also be used to deliver vectors. Another approach to targeted gene delivery is the use of molecular conjugates, which consist of protein or synthetic ligands to which a nucleic acid-or DNAbinding agent has been attached for the specific targeting of nucleic acids to cells (Cristiano et al., 1993).

*In vivo* targeting of mammalian liver sinusoidal endothelial cells has been done using hyaluronan-coated nanocapsules, generated using dispersion atomization, comprising a transposon-based vector (Kren et al. (2009) J. Clin Invest. 119:2086-99). Another approach is to use a transposon-based vector complexed to polyethyleneimine (PEI) that is delivered via intravenous injection (Liu et al. (2006) Mol Ther 13:1006-15). Merlin et al. (2017. Mol Ther. 25:1815-1830) reported lentiviral vector (LV)-mediated gene therapy targeting LSECs cells in mice via intravascular injection of the lentiviral vector.

Introduction of the nucleic acid expression cassette or the vector into endothelial cells of the subject can also be carried out *in vitro* or *ex vivo.* In embodiments, desired target enodothelial cells are removed from the subject, transfected or transduced with the nucleic acid expression cassette or the vector and reintroduced into the subject (*ex vivo* gene therapy). Alternatively, syngeneic or xenogeneic endothelial cells can be used where those cells will not generate an inappropriate immune response in the subject. Non-limiting examples of suitable target endothelial cells are endothelial cell progenitors, blood outgrowth endothelial cells (BOECs) or saphenous vein endothelial cells.

Suitable methods for the transfection or transduction and reintroduction of transfected or transduced cells into a subject are known in the art. For example, endothelial cells can be transfected or transduced *in vitro* by combining the nucleic acid expression cassette or the vector with the endothelial cells, e.g., in appropriate media, and screening for those cells harboring the DNA of interest using conventional techniques such as Southern blots and/or PCR, or by using selectable markers. Transfected or transduced cells can then be formulated into a pharmaceutical composition, and the composition introduced into the subject by various techniques, such as by intramuscular, intravenous, subcutaneous and intraperitoneal injection, or via graft implantation.

Exemplary diseases and disorders that may benefit from gene therapy, in particular endothelium-targeted gene therapy, using the nucleic acid regulatory elements, the nucleic acid expression cassettes, the vectors, or the pharmaceutical compositions described herein include, without limitation, cardiovascular diseases and disorders (e.g. coronary heart disease (CHD), heart failure (HF), deep vein thrombosis, pulmonary embolism, hypertension including pulmonary hypertension, vein graft neointima formation, ischemic or hemorrhagic stroke, myocardial ischemia and peripheral artery disease), diseases and disorders associated with endothelial cell dysfunction (e.g. corneal endothelial disorder, Fabry disease, Alzheimer's disease, Crohn's disease, Von-Hippel-Lindau disease, vascular abnormalities, vascular malformations), blood clotting or coagulation disorders (e.g. hemophilia A, hemophilia B or von Willebrand's disease), diabetes, insulin resistance, cancer, vascular inflammation, kidney disease, vascular leakage, hemorrhagic fever, and autoimmune disease.

Certain diseases and disorders as described herein may be associated with (e.g. causing or resulting from) an endothelial cell dysfunction and benefit from endothelium-directed gene therapy as described herein e.g. through the expression of an appropriate therapeutic protein (e.g. a structural therapeutic protein or an enzyme or a transcription factor) or an appropriate non-coding RNA (e.g. siRNA, microRNA, lncRNA or circRNA) in the endothelial cell. Other diseases and disorder as described herein, such as, without limitation, coagulation disorders (e.g. hemophilia A and B, FVII deficiency, von Willebrand's disease), diabetes, α1-antitrypsin deficiency and kidney failure, may benefit from endothelium-directed gene therapy as described herein through expression of a secretable therapeutic protein as described herein from the endothelial cell.

A further aspect relates to the nucleic acid regulatory elements, the nucleic acid expression cassettes, the vectors, or the pharmaceutical compositions described herein for use in the treatment of a disease or disorder selected from the group comprising: a cardiovascular disease or disorder (e.g. coronary heart disease (CHD), heart failure (HF), deep vein thrombosis, pulmonary embolism, hypertension including pulmonary hypertension, vein graft neointima formation, ischemic or hemorrhagic stroke, myocardial ischemia and peripheral artery disease), a disease or disorder associated with endothelial cell dysfunction (e.g. corneal endothelial disorder, Fabry disease, Alzheimer's disease, Crohn's disease, Von-Hippel-Lindau disease, vascular abnormalities, vascular malformations), a blood clotting or coagulation disorder (e.g. hemophilia A, hemophilia B or von Willebrand's disease), diabetes, insulin resistance, cancer, vascular inflammation, kidney disease, vascular leakage, hemorrhagic fever, and autoimmune disease.

A related aspect is directed to use of the nucleic acid regulatory elements, the nucleic acid expression cassettes, the vectors, or the pharmaceutical compositions described herein for the manufacture of a medicament for the treatment of a disease or disorder selected from the group comprising: a cardiovascular disease or disorder (e.g. coronary heart disease (CHD), heart failure (HF), deep vein thrombosis, pulmonary embolism, hypertension including pulmonary hypertension, vein graft neointima formation, ischemic or hemorrhagic stroke, myocardial ischemia and peripheral artery disease), a disease or disorder associated with endothelial cell dysfunction (e.g. corneal endothelial disorder, Fabry disease, Alzheimer's disease, Crohn's disease, Von-Hippel-Lindau disease, vascular abnormalities, vascular malformations), a blood clotting or coagulation disorder (e.g. hemophilia A, hemophilia B or von Willebrand's disease) diabetes, insulin resistance, cancer, vascular inflammation, kidney disease, vascular leakage, hemorrhagic fever, and autoimmune disease.

A related aspect is directed to a method for treating a disease or disorder selected from the group comprising: a cardiovascular disease or disorder (e.g. coronary heart disease (CHD), heart failure (HF), deep vein thrombosis, pulmonary embolism, hypertension including pulmonary hypertension, vein graft neointima formation, ischemic or hemorrhagic stroke, myocardial ischemia and peripheral artery disease), a disease or disorder associated with endothelial cell dysfunction (e.g. corneal endothelial disorder, Fabry disease, Alzheimer's disease, Crohn's disease, Von-Hippel-Lindau disease, vascular abnormalities, vascular malformations), a blood clotting or coagulation disorder (e.g. hemophilia A, hemophilia B or von Willebrand's disease) diabetes, insulin resistance, cancer, vascular inflammation, kidney disease, vascular leakage, hemorrhagic fever, and autoimmune disease, in a subject in need of said treatment, said method comprising administering a therapeutically effective amount of the nucleic acid expression cassettes, the vectors, or the pharmaceutical compositions described herein to the subject.

In embodiments, the disease or disorder is a cardiovascular disease or disorder selected from coronary heart disease (CHD), heart failure (HF), deep vein thrombosis, pulmonary embolism, hypertension including pulmonary hypertension, vein graft neointima formation, ischemic or hemorrhagic stroke, myocardial ischemia and peripheral artery disease.

In embodiments, the disease or disorder is a disease or disorder associated with endothelial cell dysfunction selected from corneal endothelial disorder, Fabry disease, Alzheimer's disease, Crohn's disease, Von-Hippel-Lindau disease, vascular abnormalities or vascular malformations.

In embodiments, the disease or disorder is a blood clotting disorder selected from hemophilia A, hemophilia B or von Willebrand's disease.

According to a specific embodiment, the therapeutic protein encoded by the transgene in the nucleic acid expression cassette or the vector is factor VIII, and the disease or disorder to be treated is hemophilia A.

As used herein, a phrase such as "a subject in need of treatment" includes subjects that would benefit from treatment of a recited disease or disorder. Such subjects may include, without limitation, those that have been diagnosed with said disease or disorder, those prone to contract or develop said disease or disorder and/or those in whom said disease or disorder is to be prevented.

The terms "subject" and "patient" are used interchangeably herein and refer to animals, preferably vertebrates, more preferably mammals, and specifically include human patients and non-human mammals. "Mammalian" subjects include, but are not limited to, humans, domestic animals, commercial animals, farm animals, zoo animals, sport animals, pet and experimental animals such as dogs, cats, guinea pigs, rabbits, rats, mice, horses, cattle, cows; primates such as apes, monkeys, orang-utans, and chimpanzees; canids such as dogs and wolves; felids such as cats, lions, and tigers; equids such as horses, donkeys, and zebras; food animals such as cows, pigs, and sheep; ungulates such as deer and giraffes; rodents such as mice, rats, hamsters and guinea pigs; and so on. Preferred patients or subjects are human subjects.

A 'therapeutic amount' or 'therapeutically effective amount' as used herein refers to the amount of (trans)gene product effective to treat a disease or disorder in a subject, i.e., to obtain a desired local or systemic effect. The term thus refers to the quantity of (trans)gene product that elicits the biological or medicinal response in a tissue, system, animal, or human that is being sought by a researcher, veterinarian, medical doctor or other clinician. Such amount will typically depend on the (trans)gene product and the severity of the disease, but can be decided by the skilled person, possibly through routine experimentation.

Typically, the amount of (trans)gene product expressed when using an expression cassette or vector as described herein (i.e., with at least one endothelium-targeted nucleic acid regulatory element as described herein) is higher than when an identical expression cassette or vector is used but without such nucleic acid regulatory element therein. The expression may be at least double as high, at least five times as high, at least ten times as high, at least 20 times as high, at least 30 times as high, at least 40 times as high, at least 50 times as high, or even at least 60 times as high as when compared to the same nucleic acid expression cassette or vector without an endothelium-targeted nucleic acid regulatory element as described herein. Moreover, the higher expression preferably remains targeted to endothelial cells or tissue. Furthermore, the expression cassettes and vectors described herein can direct the expression of a therapeutic amount of the (trans)gene product for an extended period. Typically, (trans)gene expression is envisaged to last at least 20 days, at least 50 days, at least 100 days, at least 200 days, and in some instances 300 days or more. Expression of the (trans)gene product (e.g. polypeptide) can be measured by any art-recognized means, such as by antibody-based assays, e.g. a Western Blot or an ELISA assay, for instance to evaluate whether therapeutic expression of the (trans)gene product is achieved. Expression of the (trans)gene product may also be measured in a bioassay that detects an enzymatic or biological activity of the (trans)gene product.

Further disclosed herein is the use of a nucleic acid expression cassette or a vector disclosed herein for expressing a transgene product in an endothelial cell, wherein the nucleic acid expression cassette or the vector comprises a nucleic acid regulatory element disclosed herein operably linked to a promoter and a transgene, in particular by transfecting or transducing the endothelial cell with the nucleic acid expression cassette or the vector.

Further disclosed herein is a method for expressing a transgene product in an endothelial cell comprising:
- transfecting or transducing the endothelial cell with a nucleic acid expression cassette or a vector disclosed herein, wherein the nucleic acid expression cassette or the vector comprises a nucleic acid regulatory element disclosed herein operably linked to a promoter and a transgene; and
- expressing the transgene product in the endothelial cell.

In embodiments, expression of the transgene product in the endothelial cell may comprise culturing the endothelial cell under suitable conditions to allow expression of the transgene product in the endothelial cell.

In further embodiments, the method may further comprise a step of recovering the transgene product from the endothelial cell and/or the culture medium.

In particular embodiments, the endothelial cell is a liver sinusoidal endothelial cell. In particular embodiments, the endothelial cell is a cardiac endothelial cell.

Non-viral transfection or viral vector-mediated transduction of endothelial cells may be performed *in vitro, ex vivo* or *in vivo.* The *in vitro* approach requires the *in vitro* transfection or transduction of endothelial cells, e.g. endothelial cells previously harvested from a subject, endothelial cell lines or endothelial cells differentiated from e.g. induced pluripotent stem cells or embryonic cells. The *ex vivo* approach requires harvesting of endothelial cells from a subject, *in vitro* transfection or transduction, and optionally re-introduction of the transfected or transduced endothelial cells into the subject. The *in vivo* approach requires the administration of the nucleic acid expression cassette or the vector disclosed herein toa subject. In embodiments, the transfection or transduction of the endothelial cells is performed *in vitro* or *ex vivo.*

The present inventors have shown high levels of transgene expression in liver sinusoidal endothelial cells (LSECs) after *in vivo* transduction with an AAV vector comprising a cardiac vascular endothelial cell-derived cis-regulatory element (CV-EC-CRE) as described herein and/or an endothelial cell-targeted cis-regulatory element from the prior art comprising or consisting of SEQ ID NO:17, operably linked to a promoter and a transgene. This may be advantageous to optimize (trans)gene expression in the liver, more particularly in LSECs. Maximizing (trans)gene expression in LSCEs may be particularly advantageous for the treatment of hemophilia A since the FVIII protein is naturally produced by LSECs (Shahani et al. 2014. J Thromb Haemost. 12:36-42).

Accordingly, also disclosed herein is a method, such as an *in vivo* method or an *ex vivo* or *in vitro* method, for expressing a transgene product in a liver sinusoidal endothelial cell, said method comprising:
- introducing into the liver sinusoidal endothelial cell (A) an AAV vector comprising at least one, such as one or two or more, nucleic acid regulatory element operably linked to a promoter and a transgene, wherein said nucleic acid regulatory element comprises, consists essentially of or consists of the sequence set forth in SEQ ID NO:17, a sequence having at least 80%, preferably at least 85%, more preferably at least 90%, even more preferably at least 95%, such as 95%, 96%, 97%, 98%, or 99%, identity to (the full-length of) SEQ ID NO:17, or a functional fragment thereof as described herein; (B) an AAV vector comprising at least one, such as one or two or more, nucleic acid regulatory element operably linked to a promoter and a transgene, wherein said nucleic acid regulatory element comprises, consists essentially of, or consists of a sequence selected from the group consisting of: SEQ ID NO:3, SEQ ID NO:1, SEQ ID NO:2, and a sequence having at least 80%, preferably at least 85%, more preferably at least 90%, even more preferably at least 95%, such as 95%, 96%, 97%, 98%, or 99%, identity to (the full-length of) any one SEQ ID NO:3, SEQ ID NO: 1 or SEQ ID NO:2, or a functional fragment thereof as described herein; or (C) an AAV vector comprising at least one nucleic acid regulatory element comprising, consisting essentially of, or consisting of a sequence selected from the group consisting of: SEQ ID NO:3, SEQ ID NO:1, SEQ ID NO:2, and a sequence having at least 80%, preferably at least 85%, more preferably at least 90%, even more preferably at least 95%, such as 95%, 96%, 97%, 98%, or 99%, identity to (the full-length of) any one SEQ ID NO:3, SEQ ID NO:1 or SEQ ID NO:2, or a functional fragment thereof as described herein and at least one nucleic acid regulatory element comprising, consisting essentially of or consisting of the sequence set forth in SEQ ID NO: 17, a sequence having at least 80%, preferably at least 85%, more preferably at least 90%, even more preferably at least 95%, such as 95%, 96%, 97%, 98%, or 99%, identity to (the full-length of) SEQ ID NO:17, or a functional fragment thereof as described herein, wherein said nucleic acid regulatory elements are operably linked to a promoter and a transgene; and
- expressing the transgene product in the liver sinusoidal endothelial cell.

Further disclosed herein is an AAV vector comprising at least one, such as one or two or more, nucleic acid regulatory element operably linked to a promoter and a transgene, for use in liver-directed gene therapy, wherein said AAV vector is introduced in a liver sinusoidal endothelial cell of the subject, and wherein said nucleic acid regulatory element comprises, consists essentially of or consists of the sequence set forth in SEQ ID NO:17, a sequence having at least 80%, preferably at least 85%, more preferably at least 90%, even more preferably at least 95%, such as 95%, 96%, 97%, 98%, or 99%, identity to (the full-length of) SEQ ID NO: 17, or a functional fragment thereof as described herein. Also disclosed herein is an AAV vector comprising at least one, such as one or two or more, nucleic acid regulatory element operably linked to a promoter and a transgene, for use in liver-directed gene therapy, wherein said AAV vector is introduced in a liver sinusoidal endothelial cell of the subject, and wherein said nucleic acid regulatory element comprises, consists essentially of, or consists of a sequence selected from the group consisting of: SEQ ID NO:3, SEQ ID NO: 1, SEQ ID NO:2, and a sequence having at least 80%, preferably at least 85%, more preferably at least 90%, even more preferably at least 95%, such as 95%, 96%, 97%, 98%, or 99%, identity to (the full-length of) any one SEQ ID NO:3, SEQ ID NO: 1 or SEQ ID NO:2, or a functional fragment thereof as described herein. Also disclosed herein is an AAV vector for use in leverdirected gene therapy, wherein said AAV vector is introduced in a liver sinusoidal endothelial cell of the subject, and wherein said AAV vector comprises at least one nucleic acid regulatory element comprising, consisting essentially of, or consisting of a sequence selected from the group consisting of: SEQ ID NO:3, SEQ ID NO:1, SEQ ID NO:2, and a sequence having at least 80%, preferably at least 85%, more preferably at least 90%, even more preferably at least 95%, such as 95%, 96%, 97%, 98%, or 99%, identity to (the full-length of) any one SEQ ID NO:3, SEQ ID NO:1 or SEQ ID NO:2, or a functional fragment thereof as described herein and at least one nucleic acid regulatory element comprising, consisting essentially of or consisting of the sequence set forth in SEQ ID NO:17, a sequence having at least 80%, preferably at least 85%, more preferably at least 90%, even more preferably at least 95%, such as 95%, 96%, 97%, 98%, or 99%, identity to (the full-length of) SEQ ID NO:17, or a functional fragment thereof as described herein, wherein said nucleic acid regulatory elements are operably linked to a promoter and a transgene.

Also disclosed herein is a method for liver-directed gene therapy in a subject in need of said gene therapy, said method comprising:
- introducing in a liver sinusoidal endothelial cell of the subject (A) an AAV vector comprising at least one, such as one or two or more, nucleic acid regulatory element operably linked to a promoter and a transgene, wherein said nucleic acid regulatory element comprises, consists essentially of or consists of the sequence set forth in SEQ ID NO:17, a sequence having at least 80%, preferably at least 85%, more preferably at least 90%, even more preferably at least 95%, such as 95%, 96%, 97%, 98%, or 99%, identity to (the full-length of) SEQ ID NO:17, or a functional fragment thereof as described herein, (B) an AAV vector comprising at least one, such as one or two or more, nucleic acid regulatory element operably linked to a promoter and a transgene, wherein said nucleic acid regulatory element comprises, consists essentially of, or consists of a sequence selected from the group consisting of: SEQ ID NO: 3, SEQ ID NO: 1, SEQ ID NO: 2, and a sequence having at least 80%, preferably at least 85%, more preferably at least 90%, even more preferably at least 95%, such as 95%, 96%, 97%, 98%, or 99%, identity to (the full-length of) any one SEQ ID NO:3, SEQ ID NO:1 or SEQ ID NO:2, or a functional fragment thereof as described herein, or (C) an AAV vector comprising at least one nucleic acid regulatory element comprising, consisting essentially of, or consisting of a sequence selected from the group consisting of: SEQ ID NO:3, SEQ ID NO: 1, SEQ ID NO:2, and a sequence having at least 80%, preferably at least 85%, more preferably at least 90%, even more preferably at least 95%, such as 95%, 96%, 97%, 98%, or 99%, identity to (the full-length of) any one SEQ ID NO:3, SEQ ID NO:1 or SEQ ID NO:2, or a functional fragment thereof as described herein and at least one nucleic acid regulatory element comprising, consisting essentially of or consisting of the sequence set forth in SEQ ID NO:17, a sequence having at least 80%, preferably at least 85%, more preferably at least 90%, even more preferably at least 95%, such as 95%, 96%, 97%, 98%, or 99%, identity to (the full-length of) SEQ ID NO:17, or a functional fragment thereof as described herein, wherein said nucleic acid regulatory elements are operably linked to a promoter and a transgene; and
- expressing a therapeutically effective amount of the transgene product in the liver sinusoidal endothelial cell of the subject.

Also disclosed herein is a method of treating hemophilia A in a subject in need thereof, said method comprising performing the method for expressing a transgene product in a liver sinusoidal endothelial cell as described herein.

Also disclosed herein is a method of treating hemophilia A in a subject in need thereof, said method comprising performing the liver-directed gene therapy method as described herein.

Preferably, the AAV vector may comprise at least one, such as one or two or more, nucleic acid regulatory element comprising, consisting essentially of, or consisting of a sequence selected from the group consisting of: SEQ ID NO:3, SEQ ID NO:1, SEQ ID NO:2, and a sequence having at least 80%, preferably at least 85%, more preferably at least 90%, even more preferably at least 95%, such as 95%, 96%, 97%, 98%, or 99%, identity to (the full-length of) any one SEQ ID NO:3, SEQ ID NO:1 or SEQ ID NO:2, or a functional fragment thereof as described herein; and a nucleic acid regulatory element comprising, consisting essentially of or consisting of the sequence set forth in SEQ ID NO:17, a sequence having at least 80%, preferably at least 85%, more preferably at least 90%, even more preferably at least 95%, such as 95%, 96%, 97%, 98%, or 99%, identity to (the full-length of) SEQ ID NO:17, or a functional fragment thereof as described herein. In particular, the AAV vector may comprise a nucleic acid regulatory element comprising, consisting essentially of, or consisting of the sequence set forth in SEQ ID NO:3; and a nucleic acid regulatory element comprising, consisting essentially of, or consisting of the sequence set forth in SEQ ID NO:17. The AAV vector may comprise a nucleic acid regulatory element comprising, consisting essentially of, or consisting of the sequence set forth in SEQ ID NO:1; and a nucleic acid regulatory element comprising, consisting essentially of, or consisting of the sequence set forth in SEQ ID NO:17. The AAV vector may comprises a nucleic acid regulatory element comprising, consisting essentially of, or consisting of the sequence set forth in SEQ ID NO:2; and a nucleic acid regulatory element comprising, consisting essentially of, or consisting of the sequence set forth in SEQ ID NO: 17. The nucleic acid regulatory elements in the AAV vectors can be combined in tandem or they can be combined with one or more intervening or flanking nucleotides (e.g. nucleotides used for cloning purposes) between one or more of the regulatory elements. Advantageously, the combination of a CV-EC-CRE as described herein and the prior art EC-CRE led to a synergistic increase in transgene expression in LSECs following *in vivo* AAV transduction.

The promoter in the AAV vector may be tissue-specific or ubiquitously expressed. Preferably, the promoter is an endothelium-targeted promoter as described elsewhere herein. For example, the promoter may be an endothelium-targeted promoter selected from the group consisting of: an ICAM2 promoter, a FVIII promoter, and an EDN1 promoter, preferably an ICAM2 promoter.

Preferably, the transgene in the AAV vector encodes a therapeutic protein. Preferably, the transgene in the AAV vector encodes a coagulation factor VIII as described elsewhere herein.

The AAV vector may further contain an intron and/or a polyadenylation signal as described elsewhere herein.

The AAV vector may be an AAV9 vector.

It is understood by the skilled person that the use of the nucleic acid regulatory elements, the nucleic acid expression cassettes and vectors disclosed herein has implications beyond gene therapy, e.g. coaxed differentiation of stem cells into endothelial cell precursors or endothelial cells, transgenic models for over-expression of proteins in endothelial cells or their precursors, etc.

It is to be understood that although particular embodiments, specific constructions and configurations, as well as materials, have been discussed herein for methods and applications according to the present invention, various changes or modifications in form and detail may be made without departing from the scope and spirit of this invention.

The following examples are provided to better illustrate particular embodiments, and they should not be considered limiting the application. The application is limited only by the claims.

### EXAMPLES

### Example 1: Identification of normal human coronary artery endothelial cells (CAEC) & cardiac microvascular endothelial cells (CMEC)-derived nucleic acid regulatory elements (designated as Cardiac Vascular Endothelial Cell-derived Cis-Regulatory Elements (CV-EC-CREs))

### Materials and methods

RNA of normal human coronary artery endothelial cells (CAEC) from four normal individuals and normal human cardiac microvascular endothelial cells (CMEC) from 4 normal individuals were purchased from SanBio.

Expression analysis in the normal human CAEC and CMEC were determined by RNA sequencing (RNAseq) (BGI tech solutions Ltd., Hong Kong). FPKM (i.e. fragments per kilobase of exon model per million reads mapped) reads correspond to normalised gene expression levels. The top 4 highly expressed genes based on RNA-sequencing analysis derived from the normal human CAEC and CMEC were selected for CRE identification: TMSB10, SERPINE1, CTGF and PTMA.

The transcriptional start sites of the selected genes were mapped using the UCSC Genome Brower Database. The nucleic acid elements were selected based on: i) the presence of DNAse hypersensitivity sites; ii) high content of epigenetic markers associated with open chromatin; iii) high content of transcriptional factor binding sites (TFBS).

### Results

Based on the aforementioned criteria, nucleic acid regulatory elements (designated as Cis-Regulatory Elements (CREs)) were selected that are associated with high expression in cardiac vascular endothelial cells (designated as CV-EC-CREs) (Table 1). The location, sequence and the length of each of the CRE is also provided (Table 1).

**Table 1: Identification of CV-EC-CREs. Location is based on the hg19 genome browser database.**

| Name | Gene | Location^{∗} | Size (bp) | SEQ ID NO: | Sequence |
|---|---|---|---|---|---|
| CV-EC-CRE3 | TMSB10 | **>hgl9_dna range=chr2:851 32272-85132697 5'pad=0 3'pad=0 strand=+** | 426 | 1 | CCCTAAGGAGAGGAAAAGTGGGGGAGGGAAGG GGACTCTGGAGGAGCAAAAGCAAGAGGCCTGG GGGCGACTGGGGTGGGGCGTCTCCTAGAGCGAG GATTGCTGCAGAGTGCGCGTGCACGCCCGCACAT CCACACCCACGGCTACACATCATATCTGCACACG CACAAAACCACACACCCTCCCACGCCGGTCCCAT ATCCTTTCTGCGGGGTGAGCAGCAGATTCCCGGA AAGGGGGAAATGTAGCCGCAAAAGCAAAGCCG GGGGCCTGGCGCCCACTCTGGCGGCGACTACAG CCCCCACGCTGGGCACACTGCGAGCTGGGGGTG CCCGGCGCGGGAGAAGCCGCGCCCAGCCGGGCT GGTAGCCCAGCGGGGGGCGCGGCAGGGCGAGG AGCAGCCCGCGCTGCTCCCGCCCCTCGGGT |
| CV-EC-CRE9 | SERPINE1 | **>hgl9_dna range=chr7:100 765690-100766211 5'pad=0 3'pad=0 strand=+** | 522 | 2 | AGGCGGGAGGTCAGGGGGCGATGGGTGGTGGG AAGGCCAGTGTCCACCCAGGGCCCACCAGTGGG TGGGGGACAGAGGGACAGTCATGGAAAATCTG AGTGTGATCTACAGTCACGAACCACCTGCTCAGG GACAGATGGAGACATCTTTCCCAGTAGCTGGAAT CAGAACTTTCCCCTGAAATCCACAGCCCGGTAAC TCACTCTCTTATCTCTGACCCTCCGGAAGCCAGAG ATCTAGCTATTTCATCAGCATGGACTTTTCTTTCT GTGCCCGTGTGACCCCTTACAAAAGAGCTCAAGC TGGAAAGAGAGGATTGGATCAAGAAAGAGAGA ACTTAGGATTCAGGGGAAAAGGGGAAAGTGAT AACCCCTTGAGTCAGATTCTGAGAAGAGGGTTTC TCTGAGAGGTCGGTGGGGAGCAGCCCGCAGGCA GCTGGACCCTGGTATGGAAGGGCTCGGCTCCTA AGGCAGGTAAGAGAAGGCAGGGCCACGTGCAT GAAGTAGAGGCAGGGGTCATGGC |
| CV-EC-CRE18 | PTMA | **>hgl9_dna range=chr2:232 571728-232572135 5'pad=0 3'pad=0 strand=+** | 408 | 3 | TCCGGGTTTTCCCCATTCCCAGCTCGGCTGCAACC GAGAGACAGACGGAAGAAACTGCCAATGGAAG GACGCAAAGCGTCAACTTTGTGTTCGCCCGCCAC TCTCGGCCAATGTGCGCTCGTGTCTCCGGAGTCC CGCCTTCCGTGTCGGGCGCCCCCTTTTGGCAGCA CAGGGACGAACCCTACGTCTCGCCATCTTGTGTG TGGCACAGGTAAATTTCTGGACTATCTTTCCACCT ATTCTGGTCCTTTTTCCCACACAACCGATTTCTTA GTTCGACAGCTAAGTGCGGTGGCGATAAGGCCA GCGTAGCCTGTTGTTCTCGGATCGCCCACGTGAG CTAGATCGCTACGTCACGCCACGCTTGCCGAGCC CGGCGCCATCTTGAGTGTGGCGTTTTGATGCTCC |
| CV-EC-CRE16 | CTGF | **>hgl9_dna range=chr6:132 275276-132275621 5'pad=0 3'pad=0 strand=+** | 346 | 16 | AGACACCCAAGACATTCACAGACTCAGAAGTA TGTTTTTATTGTGATATGGACCATCAAATGCC AGGAAACTCAAAGGGAAAGGCTGATACTCCAT CCATAATTCATGCCATGGGATCTGAATTCTTG CACTATTATCAATGACTCAGCAAATGTTTGTG TGTGCCTGGGCGGTAAAGTGGGAAATAGTGGC TTCTTATAACTTCATCCAACTTTGACTTACCC CAGCCCCTTTGATTGACAGCAGGTCAGAGTTG GGAGAAAAAGATCTTTGAATTTGTGTGCAAAA TGGTTAAGATGCACTAATTTTGACTTGTAAGC AAGTATTTATCTAGCAAATGCTTTTT |

### Example 2: In vivo validation of the identified CV-EC-CREs

### Materials and methods

### Cloning of CV-EC-CREs into AA V vectors

The CV-EC-CREs indicated in Table 1 were individually screened for their ability to enhance gene expression in an artificial synthetic context which is distinct from its natural genomic context. To do this, the CV-EC-CREs (Table 1) were first synthesized by conventional oligonucleotide synthesis, flanked with AscI restriction site at the 5' end and Acc65I at the 3' end for convenient cloning.

These synthetic CV-EC-CREs were then restricted with AscI and Acc65I and cloned into the AAV2 vector plasmid backbone (SEQ ID NO: 4) restricted with MluI at the 5' end and Acc65I at the 3' end. This cloning was possible since MluI/AscI generate compatible ends. The different CV-EC-CREs (SEQ ID NO: 1, 2, 3 and 16) (Table 1) were cloned upstream of the IFI27-EC-CRE1b cis-regulatory element in conjunction with the endothelial cell directed promoter ICAM2 (SEQ ID NO:18) in the context of a single stranded (ss) adeno-associated viral vector (AAV) backbone (Fig. 1B). The IFI27-EC-CRE1b element was previously identified and is described in patent application WO 2017/109039 A1 (SEQ ID NO:17). The ICAM2 promoter was used to drive expression of the luciferase reporter gene (*Luc2*). A synthetic polyadenylation site (SEQ ID NO:22) was cloned downstream of the *Luc2* gene to ensure adequate transcriptional termination. All AAV vectors contained an MVM (minute virus of mouse) intron (SEQ ID NO:21) upsteam of *Luc2* to enhance expression. The resulting CV-EC-CRE AAV vectors were designated as AAVss-CV-EC-CRE3-IFI27-EC-CRE1b-ICAM2-Luc-pA (SEQ ID NO: 5), AAVss-CV-EC-CRE9-IFI27-EC-CRE1b-ICAM2-Luc-pA (SEQ ID NO: 6), AAVss-CV-EC-CRE18-IFI27-EC-CRE1b-ICAM2-Luc-pA (SEQ ID NO: 7) and AAVss-CV-EC-CRE16-IFI27-EC-CRE1b-ICAM2-Luc-pA (SEQ ID NO: 15).

Similar vector designs but using different endothelial cell directed promoters such as the human FVIII promoter (SEQ ID NO:19) and the EDN1 promoter (SEQ ID NO:20) were also generated. AAVss-(CV-EC-CRE-18)-(IFI27-EC-CRE1b)-FVIIIpromoter-Luc-pA (SEQ ID NO:8) contained a combination of the regulatory elements CV-EC-CRE18 and IFI27-EC-CRE1b and a FVIII promoter to drive *Luc2.* The FVIII promoter was first synthesized with flanking restriction enzymes SpeI and NheI to generate an SpeI-FVIIIpromoter-NheI insert for convenient cloning into an SpeI-NheI restricted ssAAV vector (i.e. AAVss-CV-EC-CRE18-IFI27-EC-CRE1b-ICAM2-Luc-pA (SEQ ID NO:7) that contained the regulatory elements CV-EC-CRE-18 and IFI27-EC-CRE1. Consequently, the ICAM2 promoter was replaced with the FVIII promoter to obtain the AAVss-(CV-EC-CRE-18)-(IFI27-EC-CRE1b)-FVIIIpromoter-Luc-pA vector (SEQ ID NO:8). Similarly, AAVss-(CV-EC-CRE-18)-(IFI27-EC-CRE1b)-EDN1-Luc-pA (SEQ ID NO:9) contained a combination of CV-EC-CRE18 and IFI27-EC-CRE1b and an EDN1 promoter to drive Luc2. To clone this vector containing the EDN1 promoter, the ICAM2 promoter from AAVss-CV-EC-CRE18- IFI27-EC-CRE1b-ICAM2-Luc-pA (SEQ ID NO:7) was replaced with the EDN1 promoter. The strategy involved generating an insert fragment (IFI27-EC-CRE1b-EDN1-MVM-Luc) flanked with enzymes Acc65I and BbsI and replace the deleted fragment IFI27-EC-CRE1b-ICAM2-MVM-Luc from the vector or SEQ ID NO:7 by restriction with enzymes Acc65I and BbsI.

### Construction of AAV control vectors

Control AAV vectors devoid of the IFI27-EC-CRE1b element were also generated by deleting IFI27-EC-CRElb with Acc65I and BsiWI restriction enzymes and then religated to generate AAVss-CV-EC-CRE18-ICAM2-Luc-pA (SEQ ID NO: 10) (Fig. 1C). This vector was used as a control vector to determine if addition of CV-EC-CRE led to enhancement of gene expression.

A second control AAV vector that was devoid of both endothelial CREs (CV-EC-CRE and IFI27-EC-CRE1b) was also constructed, which contains the ICAM2 promoter driving the reporter *Luc2* gene. This control vector was designated as AAVss-ICAM2-Luc-pA (SEQ ID NO:11) (Fig. 1A). This control vector was used to determine if the combination of the endothelial CRE (CV-EC-CRE) and IFI27-EC-CRE1b led to further enhancement of gene expression compared to when only one EC-CRE was used.

Control vector AAVss-FVIIIpromoter-Luc-pA (SEQ ID NO:14) (a control AAV9 vector devoid of any CRE) was obtained by first synthesizing the FVIII promoter with flanking restriction enzymes SpeI and NheI to generate SpeI-FVIIIpromoter-NheI insert for convenient cloning into an Spe/NheI-restricted ssAAV vector, devoid of CRE, that contained the MVM intron Luc2 gene and synthetic pA.

### AA V production and titration

Plasmid maxi preps were performed to extract the AAV plasmid constructs using the standard Qiagen extraction method. The extracted plasmids were subsequently used for AAV vector production which was outsourced to SignaGen Laboratories (Maryland, US). The vectors were produced using the AAV serotype 9 capsid (abbreviated as AAV9). The vector titers were determined by quantitative real-time polymerase chain reaction (qRT-PCR) with SYBR Green using luciferase-specific primers: forward: 5'-CCCACCGTCGTATTCGTGAG-3' (SEQ ID NO:12) and reverse: 5'-TCAGGGCGATGGTTTTGTCCC-3' (SEQ ID NO:13). Known copy numbers of the corresponding vector plasmids were used for the generation of the standard curves. The AAV9 viral vectors yielded high titers (typically 10¹² to 10¹³ vector genomes (vg)/ml).

### Animal studies: In vivo validation of CV-EC-CREs

### AA V injection

AAV vector genomes were packaged into AAV9 vector particles that were injected intravenously (i.v.) into 4-5 week old CB17-SCID mice to target endothelial cells *in vivo.* Mice were injected intravenously with a dose corresponding to 10¹¹ vector genomes (vg) per mouse; 3 mice were injected per group with AAV9 viral vectors that expressed the luciferase gene (Fig. 2) from the following AAV vectors (Fig. 1):
(1) AAVss-ICAM2-Luc-pA (a control AAV9 vector devoid of any CRE)
(2) AAVss-IFI27-EC-CRE1b-ICAM2-Luc-pA (AAV9 vector with one endothelial cis regulatory element designated as IFI27-EC-CRE1b)
(3) AAVss-(CV-EC-CRE-3)-(IFI27-EC-CRE1b)-ICAM2-Luc-pA (AAV9 vector with a combination of CV-EC-CRE3 and IFI27-EC-CRE1b).
(4) AAVss-(CV-EC-CRE-9)-(IFI27-EC-CRE1b)-ICAM2-Luc-pA (AAV9 vector with a combination of CV-EC-CRE9 and IFI27-EC-CRE1b).
(5) AAVss-(CV-EC-CRE-18)-(IFI27-EC-CRElb)-ICAM2-Luc-pA (AAV9 vector with a combination of CV-EC-CRE18 and IFI27-EC-CRE1b).

Five months post vector injection, the mice were euthanized and the livers were removed. The liver sinusoidal endothelial cells (LSECs) were isolated from the transduced liver and enriched for subsequent reporter gene expression analysis *in vitro* using a luminometer. Luciferase activity was determined by luminometric analysis.

### LSEC isolation and enrichment from mouse liver and quantification of luciferase activity

LSEC were isolated and purified according to the protocol from Miltenyi Biotec encompassing a liver dissociation step, removal of debris after dissociation and CD146+ magnetic-activated cell sorting (MACS). LSECs are lining the vasculature in the liver and separate the liver parenchyma from the blood. Typically, LSEC suspensions were obtained with >95% purity after *in vitro* enrichment by magnetic activated cell sorting (MACS) with EC-specific CD146-antibody tagged microbeads. Freshly isolated and purified LSEC were counted and 40,000, 80,000, 100,000, 200,000 and 300,000 LSEC cells were taken for measuring luciferase activity using a luminometer. Luciferase activity is expressed as relative luminescence units (RLU).

### Results

### In vivo validation CV-EC-CRE by luminometric analysis in purified LSEC

Inclusion of the regulatory elements CV-EC-CRE3, CV-EC-CRE9 or CV-EC-CRE18 as identified in example 1 increased reporter gene expression in *in vivo* transduced LSEC compared to control constructs devoid of CV-EC-CRE (such as control ICAM2-Luc or IFI27-EC-CRE1b-ICAM2-Luc vectors) (Fig. 3). The combination of CV-EC-CRE with IFI27-EC-CRE1b yielded higher luciferase gene expression levels compared to IFI27-EC-CRE1b by itself. IFI27-EC-CRE1b itself increasesd gene expression levels compared to a control construct devoid of IFI27-EC-CRE1b (ICAM2-Luc).

### Vector dose response analysis

CB17 SCID mice were injected with 2 different doses of vectors: 1×10¹¹vg and 3×10¹¹ vg per mouse and luciferase activity was measured in purified LSECs isolated from the mice livers. The results ae shown in Fig. 4A (1×10¹¹vg/mouse) and Fig. 4B (3×10¹¹ vg per mouse).

The results demonstrate that inclusion of CV-EC-CRE3, CV-EC-CRE9 and CV-EC-CRE18 increased reporter gene expression whereby higher vector doses yielded higher expression levels. CV-EC-CRE reproducibly increased gene expression levels compared to a control construct devoid of a CV-EC-CRE. About 2 to 5-fold enhancement of luciferase gene expression was observed due to the addition of a CV-EC-CRE.

### Comparative promoter analysis

Mice were injected intravenously with a dose corresponding to 8×10¹¹ vector genomes (vg) per mouse . 4 mice were injected per group with AAV9 viral vectors that expressed the luciferase gene from the following AAV vectors :
(1) AAVss-FVIIIpromoter-Luc-pA (a control AAV9 vector devoid of any CRE)
(2) AAVss-(CV-EC-CRE-18)-(IFI27-EC-CRE1b)-FVIIIpromoter-Luc-pA (AAV vector with a combination of CV-EC-CRE18 and IFI27-EC-CRE1b driven from a FVIII promoter).
(3) AAVss-(CV-EC-CRE-18)-(IFI27-EC-CRE1b)-EDN1-Luc-pA (AAV vector with a combination of CV-EC-CRE18 and IFI27-EC-CRE1b driven from the EDN1 promoter).
(4) AAVss-(CV-EC-CRE-18)-(IFI27-EC-CRElb)-ICAM2-Luc-pA (AAV vector with a combination of CV-EC-CRE18 and IFI27-EC-CRE1b driven from the ICAM2 promoter).

Four months post vector injection, the mice were subjected to euthanasia and the liver were removed. LSECs were isolated from the *in vivo* transduced livers and enriched for subsequent reporter gene expression analysis in vitro using a luminometer. Luciferase activity was determined by luminometric analysis.

Inclusion of the regulatory element CV-EC-CRE-18 in combination with the regulatory element IFI27-EC-CRE1b in the AAV vector increased reporter gene expression driven from the endothelial-targeted FVIII promoter by about 8.5-11.5 folds compared to the control vector without regulatory elements (Fig. 5). Inclusion of the combination of the regulatory elements CV-EC-CRE-18 and IFI27-EC-CRE1b operably linked to the ICAM2 promoter resulted in the highest reporter gene expression compared to the expression levels achieved by the endothelial cell-targeted promoters FVIII or EDN1 operably linked to CV-EC-CRE-18 and IFI27-EC-CRE1b (Fig. 5).

### Validation of CV-EC-CREs in isolated mice LSECs after in vivo AA V-Luc transduction

5-6 weeks old CB-17 SCID mice were injected intravenously at a dose of 1×10¹² vg per mouse (6 mice per group) with AAV9 viral vectors that expressed the luciferase gene from the following AAV vectors:
(1) AAVss-ICAM2-Luc-pA (a control AAV9 vector devoid of any CRE)
(2) AAVss-IFI27-EC-CRE1b-ICAM2-Luc-pA (AAV vector with one endothelial cis regulatory element designated as IFI27-EC-CRE1b)
(3) AAVss-CRE18-ICAM2-Luc-pA (AAV vector with one endothelial cis regulatory element designated asCRE18)
(4) AAVss-(CV-EC-CRE-3)-(IFI27-EC-CRE1b)-ICAM2-Luc-pA (AAV vector with a combination of CV-EC-CRE9 and IFI27-EC-CRE1b).
(5) AAVss-(CV-EC-CRE-9)-(IFI27-EC-CRE1b)-ICAM2-Luc-pA (AAV vector with a combination of CV-EC-CRE9 and IFI27-EC-CRE1b).
(6) AAVss-(CV-EC-CRE-16)-(IFI27-EC-CRE1b)-ICAM2-Luc-pA (AAV vector with a combination of CV-EC-CRE16 and IFI27-EC-CRE1b).
(7) AAVss-(CV-EC-CRE-18)-(IFI27-EC-CRElb)-ICAM2-Luc-pA (AAV vector with a combination of CV-EC-CRE18 and IFI27-EC-CRE1b).

3 weeks post injection, the mice were euthanized and LSECs were isolated and purified from the mouse livers. Luciferase gene expression was quantified *in vitro* using a luminometer.

Inclusion of the regulatory elements CV-EC-CRE3, CV-EC-CRE9 and CV-EC-CRE18 increased reporter *(Luc)* gene expression compared to the expression level obtained with the IFI27-EC-CRE1b-driven construct (Fig. 6A). Inclusion of the regulatory element CV-EC-CRE16 did not lead to any significant increase in *luc* expression compared to the expression level obtained with the IFI27-EC-CRE1b-driven construct (Fig. 6A).

A synergistic effect was observed with a construct containing two CREs: CV-EC-CRE18 and IFI27-EC-CRElb (Fig. 6B). Inclusion of CV-EC-CRE18 led to a 2.8-fold augmentation of *luc* gene expression; inclusion of IFI27-EC-CRE1b led to a 20.7-fold augmentation of *luc* gene expression; and their combination (CV-EC-CRE18-IFI27-EC-CRE1b) led to a 34-fold augmentation in luciferase expression when compared to the construct devoid of a CRE (ICAM2-Luc) (Fig. 6B). In conclusion, the combination of two CREs led to an unexpected synergistic enhancement of luciferase expression.

In addition to determining the luciferase activity, luciferase mRNA by quantitative reverse transcriptase polymerase chain reaction (qRT-PCR) was also measured. The results are shown in Figure 7. The observed synergistic effect was also apparent at the RNA level. Inclusion of CV-EC-CRE18 led to a 3.9-fold augmentation of luc mRNA level; unclusion of IFI27-EC-CRE1b led to a 12.7-fold augmentation; and their combination (CV-EC-CRE18-IFI27-EC-CRE1b) led to a 25-fold increase in mRNA level when compared to the construct devoid of a CRE (ICAM2-Luc). In conclusion, the combination of two CREs led to an unexpected synergistic enhancement of luciferase mRNA, consistent with the synergistic effect observed at the protein level (Fig. 6B).

### Conclusions

These data demonstrated that higher levels of transgene expression can be obtained in endothelial cells, in particular liver sinusoidal endothelial cells (LSECs) after *in vivo* transduction with an AAV vector comprising a CV-EC-CRE as identified herein operably linked to a promoter, in particular an endothelial-targeted promoter. This overcomes the limitation of more conventional vector designs based on standard endothelial-targeted promoters (such as ICAM2, EDN1, CDH5 etc.), which typically result in lower and therefore suboptimal expression levels in the desired target tissue. For example, the ICAM2, EDN1 and CDH5 endothelial- targeted promoters are relatively poorly expressed in EC *in vivo,* despite being widely considered as quintessential EC-targeted promoters.

Moreover, these novel CV-EC-CREs operably linked to an endothelial-targeted promoter such as ICAM2, but also other EC-targeted promoters (e.g. FVIII) yielded much higher expression in endothelial cells *in vivo.*

Hence, the use of these novel CV-EC-CREs constitutes an attractive strategy to maximize the overall efficacy and safety of gene therapy for gene therapy of disease that would benefit from EC-targeted transgene expression.

## Claims

1. A nucleic acid regulatory element for enhancing endothelial cell-targeted gene expression, comprising a sequence selected from the group consisting of: SEQ ID NO:3, SEQ ID NO:1, SEQ ID NO 2, and a sequence having at least 80%, preferably at least 85%, more preferably at least 90%, even more preferably at least 95%, such as 95%, 96%, 97%, 98%, or 99% identity to the full-length of any one of SEQ ID NO:3, SEQ ID NO:1 or SEQ ID NO:2, or a functional fragment thereof, wherein said nucleic acid regulatory element has a maximal length of 600 nucleotides.

2. *In vitro* or *ex vivo* use of a nucleic acid regulatory element according to claim 1 in a nucleic acid expression cassette or a vector for enhancing endothelial cell-targeted gene expression.

3. A nucleic acid expression cassette comprising at least one nucleic acid regulatory element, operably linked to a promoter and a transgene, wherein said nucleic acid regulatory element comprises a sequence selected from the group consisting of: SEQ ID NO:3, SEQ ID NO:1, SEQ ID NO:2, and a sequence having at least 80%, preferably at least 85%, more preferably at least 90%, even more preferably at least 95%, such as 95%, 96%, 97%, 98%, or 99% identity to the full-length of any one of SEQ ID NO:3, SEQ ID NO:1 or SEQ ID NO:2, or a functional fragment thereof.

4. The nucleic acid expression cassette according to claim 3, further comprising an endothelial cell-targeted regulatory element comprising SEQ ID NO:17 or a sequence having at least 95% identity to the full-length of SEQ ID NO:17.

5. The nucleic acid expression cassette according to claim 4, wherein the promoter is an endothelial cell-targeted promoter, preferably an ICAM2 promoter, a FVIII promoter or a EDN1 promoter, more preferably an ICAM2 promoter.

6. The nucleic acid expression cassette according any one of claims 3 to 5, wherein the transgene encodes a therapeutic protein.

7. The nucleic acid expression cassette according any one of claims 3 to 5, wherein the transgene encodes a non-coding RNA, preferably a non-coding RNA selected from the group comprising a microRNA, a long non-coding RNA, a circular RNA and a small interfering RNA.

8. The nucleic acid expression cassette according to any one of claims 3 to 7, further comprising an intron, preferably a Minute Virus of Mouse (MVM) intron.

9. The nucleic acid expression cassette according to any one of claims 3 to 8, further comprising a polyadenylation signal, preferably a synthetic polyadenylation signal.

10. A vector comprising the nucleic acid expression cassette according to any one of claims 3 to 9.

11. The vector according to claim 10, which is a viral vector, preferably an adeno-associated viral (AAV) vector.

12. A pharmaceutical composition comprising the nucleic acid expression cassette according to any one of claims 3 to 9, or the vector according to claim 10 or 11, and a pharmaceutically acceptable carrier.

13. The nucleic acid expression cassette according to any one of claims 3 to 9, the vector according to claim 10 or 11, or the pharmaceutical composition according to claim 12 for use in medicine.

14. The nucleic acid expression cassette according to any one of claims 3 to 9, the vector according to claim 10 or 11, or the pharmaceutical composition according to claim 12 for use in gene therapy, preferably endothelium-directed gene therapy.

15. An *in vitro* or *ex vivo* method, for expressing a transgene product in an endothelial cell, comprising:
- introducing the nucleic acid expression cassette according to any one of claims 3 to 9, or the vector according to claim 10 or 11 into the endothelial cell;
- expressing the transgene product in the endothelial cell.
